# EUROPÄISCHE PATENTSCHRIFT

(11) **EP 0 704 443 B1**
(45) Veröffentlichungstag und Bekanntmachung des Hinweises auf die Patenterteilung: **27.12.2000**
(21) Anmeldenummer: 95111579.9
(22) Anmeldetag: 24.07.1995
(51) Int. Cl.: C07D 401/10, C07D 498/04, C07D 513/06, C07D 498/16, C07D 513/16, A61K 31/47

(54) **Chinolon- und Naphthyridoncarbonsäurederivate**
Quinolone and naphthyridone carboxylic acid derivatives
Dérivés de l'acide quinolone et naphthyridonecarboxylique

(30) Priorität: 04.08.1994 DE 4427530
(43) Veröffentlichungstag der Anmeldung: 03.04.1996
(73) Patentinhaber: BAYER AG, 51368 Leverkusen (DE)
(72) Erfinder: Petersen, Uwe, Dr., D-51375 Leverkusen (DE); Schenke, Thomas, Dr., D-51469 Bergisch Gladbach (DE); Jaetsch, Thomas, Dr., D-50668 Köln (DE); Bartel, Stephan, Dr., D-51465 Bergisch Gladbach (DE); Bremm, Klaus Dieter, Dr., D-45661 Recklinghausen (DE); Endermann, Rainer, Dr., D-42113 Wuppertal (DE); Metzger, Karl Georg, Dr., D-42115 Wuppertal (DE)

(56) Entgegenhaltungen:
- EP-A- 0 343 524
- EP-A- 0 387 802
- EP-A- 0 429 304
- EP-A- 0 520 240
- EP-A- 0 523 512
- EP-A- 0 607 825
- EP-A- 0 647 644
- EP-A- 0 671 391
- WO-A-92/12146

## Beschreibung

Die Erfindung betrifft neue Chinolon- und Naphthyriconcarbonsäure-Derivate, die in 7-Stellung durch einen tricyclischen Aminrest substituiert ihre Salze, Verfahren zu ihrer Herstellung sowie diese enthaltende antibakterielle Mittel.

Es sind bereits aus den Patentanmeldungen EP 520 240 (Bayer) sowie JP 4 253 973 (Banyu) Chinoloncarbonsäuren bekannt, die in 7-Stellung durch einen bicyclischen ungesättigten Aminrest substituiert sind. Diese Verbindungen zeichnen sich durch eine hohe antibakterielle Aktivität aus. Sie besitzen aber den Nachteil, daß sie ein hohes gentoxisches Potential aufweisen, was ihren Einsatz als Arzneimittel unmöglich macht. Der Erfindung liegt daher die Aufgabe zugrunde, Verbindungen aufzufinden, die bei hoher antibakterieller Wirksamkeit eine Verringerung der gentoxischen Eigenschaften aufweisen.

Es wurde jetzt gefunden, daß die Verbindungen der Formel (I)

T-Q (I)

in welcher
- Q: einen Rest der Formeln 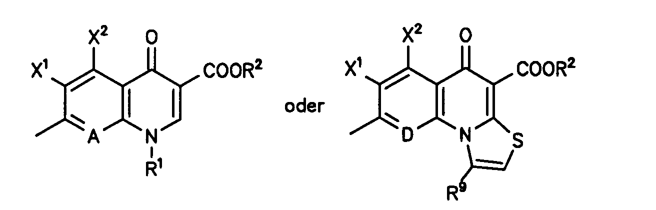 bedeutet, worin
- R¹: für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]-pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
- R²: für Wasserstoff, gegebenenfalls durch Hydroxy, Methoxy, Amino, Dimethylamino substituiertes Alkyl mit 1 bis 3 Kohlenstoffatomen, Benzyl, (5-Methyl-2-oxo-1,3-dioxol-4-yl)-methyl, Acetoxymethyl, Pivaloyloxymethyl, 5-Indanyl, Phthalidinyl oder 3-Acetoxy-2-oxo-butyl,
- R⁹: für Wasserstoff oder gegebenenfalls durch Methoxy, Hydroxy oder Halogen substituiertes Alkyl mit 1 bis 3 Kohlenstoffatomen,
- X¹: für Halogen oder Nitro,
- X²: für Wasserstoff, Halogen, Amino, Hydroxy, Methoxy, Mercapto, Methyl, Halogenmethyl oder Vinyl,
- A: für N oder C-R⁷ steht, worin
- R⁷: für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*S-CH₂-CH-CH₃, -*S-CH₂-CH-CH₂F, -*CH₂-CH₂-CH-CH₃ oder -*O-CH₂-N-R⁸, wobei das mit * markierte Atom mit dem Kohlenstoffatom von A verknüpft ist und worin
- R⁸: Wasserstoff, Methyl oder Formyl bedeutet, bilden kann, und
- D: für N oder C-R¹⁰ steht, worin
- R¹⁰: für Wasserstoff, Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
- T: einen Rest der Formel 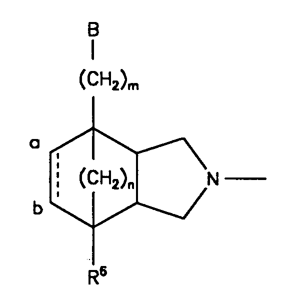 bedeutet, worin
- B: für NR³R⁴ oder OR⁵ steht, worin
- R³: für Wasserstoff, Methyl oder Alkoxycarbonyl mit 1 bis 4 Kohlenstoffatomen im Alkylteil,
- R⁴: für Wasserstoff oder Methyl und
- R⁵: für Wasserstoff oder Methyl steht,
- R⁶: für Wasserstoff oder Methyl,
- m: für 0 oder 1 und
- n: für 1 oder 2 steht,
wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali-, Silber- und Guanidiniumsalze der zugrundeliegenden Carbonsäuren bei guter Verträglichkeit eine hohe antibakterielle Wirkung insbesondere gegenüber grampositiven Bakterien aufweisen.

Bevorzugt sind die Verbindungen der Formel (I),
in welcher
- Q und T: die oben angegebene Bedeutung haben und
- R¹: für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
- R²: für Wasserstoff, Alkyl mit 1 bis 2 Kohlenstoffatomen, Benzyl oder (5-Methyl-2-oxo-1,3-dioxol-4-yl)-methyl,
- R⁹: für Wasserstoff oder gegebenenfalls durch Fluor ein- bis dreifach substituiertes Alkyl mit 1 bis 2 Kohlenstoffatomen,
- X¹: für Fluor oder Chlor,
- X²: für Wasserstoff, Halogen, Amino, Methyl, Trifluormethyl oder Vinyl,
- A: für N oder C-R⁷ steht, worin
- R⁷: für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
- R⁸: Wasserstoff oder Methyl bedeutet,
bilden kann, und
- D: für N oder C-R¹⁰ steht, worin
- R¹⁰: für Wasserstoff, Fluor, Chlor, CF₃, OCH₃ oder CH₃ steht oder auch gemeinsam mit R⁹ eine Brücke der Struktur -O-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
- B: für NR³R⁴ oder OR⁵ steht, worin
- R³: für Wasserstoff Methyl oder Alkoxycarbonyl mit 1 bis 4 Kohlenstoffatomen im Alkylteil,
- R⁴: für Wasserstoff oder Methyl und
- R⁵: für Wasserstoff oder Methyl steht,
- R⁶: für Wasserstoff oder Methyl,
- m: für 0 oder 1 und
- n: für 1 oder 2 steht,

wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I),
in welcher
- Q und T: die oben angegebene Bedeutung haben und
in welchen
- R¹: für gegebenenfalls durch Fluor ein- oder zweifach substituiertes Alkyl mit 1 bis 4 Kohlenstoffatomen, Vinyl, gegebenenfalls durch 1 Fluoratom substituiertes Cyclopropyl, gegebenenfalls durch Fluor ein- oder zweifach substituiertes Phenyl,
- R²: für Wasserstoff, Alkyl mit 1 bis 2 Kohlenstoffatomen oder (5-Methyl-2-oxo-1,3-dioxol-4-yl)-methyl,
- R⁹: für Wasserstoff oder gegebenenfalls durch Fluor ein- bis dreifach substituiertes Methyl,
- X¹: für Fluor,
- X²: für Wasserstoff, Fluor, Amino, Methyl oder Vinyl,
- A: für N oder C-R⁷ steht, worin
- R⁷: für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
- R⁸: Wasserstoff oder Methyl bedeutet,
bilden kann, und
- D: für N oder C-R¹⁰ steht, worin
- R¹⁰: für Wasserstoff, Fluor, Chlor oder OCH₃ steht oder auch gemeinsam mit R⁹ eine Brücke der Struktur -*O-CH₂-, -*N(CH₃)-CH₂-, -*N(C₂H₅)-CH₂- oder -*S-CH₂- bilden wobei das mit * markierte Atom mit dem Kohlenstoffatom von D verknüpft ist, und
- B: für NR³R⁴ oder OR⁵ steht, worin
- R³: für Wasserstoff oder Methyl,
- R⁴: für Wasserstoff oder Methyl und
- R⁵: für Wasserstoff oder Methyl steht,
- R⁶: für Wasserstoff,
- m: für 0 oder 1 und
- n: für 1 oder 2 steht,
wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali, Silber- und Guanidiniumsalze der zugrundeliegenden Carbonsäuren.

Weiterhin wurde gefunden, daß man die Verbindungen der Formel (I) erhält, wenn man Verbindungen der Fomel (II)

Y-Q (II),

in welcher
- Q: die oben angegebene Bedeutung hat und
- Y: für Halogen, inbesondere für Fluor oder Chlor steht,
mit Verbindungen der Formel (III) 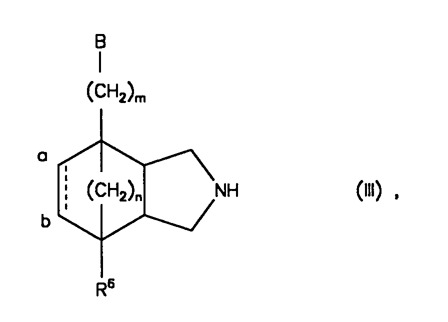 in welcher
B, R⁶, m, n, a und b die oben angegebenen Bedeutungen haben,
gegebenenfalls in Gegenwart von Säurefängern umsetzt und gegebenenfalls vorhandene Schutzgruppen abspaltet.

Verwendet man beispielsweise 6,7-Difluor-1-cyclopropyl-1,4-dihydro-8-methoxy-4-oxo-3-chinolincarbonsäure und 4-Aza-tricyclo[5.2.1.0^{2,6}]dec-8-en-1-ylamin als Ausgangsstoffe, so kann der Reaktionsverlauf durch das folgende Formelschema wiedergegeben werden:

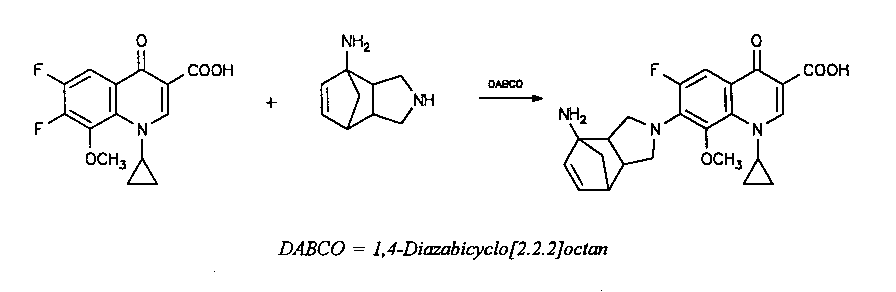

Die als Ausgangsverbindungen verwendeten Verbindungen der Formel (II) sind bekannt oder können nach bekannten Methoden hergestellt werden. Sie können sowohl als racemische als auch als enantiomerenreine Verbindungen eingesetzt werden. Als Beispiele seien genannt:
7-Chlor-1-cyclopropyl-6-fluor-1,4-dihydro-4-oxo-3-chinolincarbonsäure, 1-Cyclopropyl-6,7-difluor-1,4-dihydro-4-oxo-3-chinolincarbonsäure, 6-Chlor-1-cyclopropyl-7,8-difluor-1,4-dihydro-4-oxo-3-chinolincarbonsäure, 8-Chlor-1-cyclopropyl-6,7-difluor-1,4-dihydro-4-oxo-3-chinolincarbonsäure, 1-Cyclopropyl-6,7,8-trifluor-1,4-dihydro-4-oxo-3-chinolincarbonsäure, 5-Brom-1-cyclopropyl-6,7,8-trifluor-1,4-dihydro-4-oxo-3-chinolincarbonsäure, 5-Brom-1-(2,4-difluorphenyl)-6,7,8-trifluor-1,4-dihydro-4-oxo-3-chinolincarbonsäure, 1-Cyclopropyl-6,7-difluor-1,4-dihydro-8-methyl-4-oxo-3-chinolincarbonsäure, 6,7-Difluor-1-ethyl-1,4-dihydro-4-oxo-3-chinolincarbonsäure, 7-Chlor-6-fluor-1-ethyl-1,4-dihydro-4-oxo-3-chinolincarbonsäure,7-Chlor-6-fluor-1,4-dihydro-1-(2-hydroxyethyl)-4-oxo-3-chinolincarbonsäure,6,7-Difluor-1-(2-fluorethyl)-1,4-dihydro-4-oxo-3-chinolincarbonsäure, 7-Chlor-6-fluor-1,4-dihydro-1-methoxy-4-oxo-3-chinolincarbonsäure,7-Chlor-6-fluor-1,4-dihydro-1-methylamino-4-oxo-3-chinolincarbonsäure, 6,7-Difluor-1,4-dihydro-4-oxo-1-phenyl-3-chinolincarbonsäure, 7-Chlor-1-cyclopropyl-6-fluor-1,4-dihydro-4-oxo-1,8-naphthyridin-3-carbonsäure, 7-Chlor-1-cyclopropyl-6-fluor-1,4-dihydro-4-oxo-1,8-naphthyridin-3-carbonsäureethylester, 1-Cyclopropyl-6,7,8-trifluor-1,4-dihydro-4-oxo-3-chinolincarbonsäureethylester, 9,10-Difluor-2,3-dihydro-3-methyl-7-oxo-7H-pyrido-[1,2,3-de][1,4]benzoxacin-6-carbonsäure, 8,9-Difluor-6,7-dihydro-5-methyl-1-oxo-1H,5H-benzo[i,j]-chinolicin-2-carbonsäure, 7-Chlor-6-fluor-1-phenyl-1,4-dihydro-4-oxo-1,8-naphthyridin-3-carbonsäure, 7-Chlor-6-fluor-1-(4-fluorphenyl)-1,4-dihydro-4-oxo-1,8-naphthyridin-3-carbonsäureethylester, 6,7,8-Trifluor-1,4-dihydro-1-methylamino-4-oxo-3-chinolincarbonsäure, 1-Amino-6,7,8-trifluor-1,4-dihydro-4-oxo-3-chinolincarbonsäure, 6,7,8-Trifluor-1,4-dihydro-1-dimethylamino-4-oxo-3-chinolincarbonsäure,6,7-Difluor-1-(4-fluorphenyl)-1,4-dihydro-8-methyl-4-oxo-3-chinolincarbonsäure, 7-Chlor-6-fluor-1-(4-fluorphenyl)-1,4-dihydro-4-oxo-3-chinolincarbonsäure, 7-Chlor-6-fluor-1-(2,4-difluorphenyl)-1,4-dihydro-4-oxo-3-chinolincarbonsäure, 6,7,8-Trifluor-1-(4-fluorphenyl)-1,4-dihydro-4-oxo-3-chinolincarbonsäure, 1-Cyclopropyl-6,7-difluor-1,4-dihydro-5-methyl-4-oxo-3-chinolincarbonsäure, 7-Chlor-1-cyclopropyl-6-fluor-1,4-dihydro-5-methyl-4-oxo-1,8-naphthyridin-3-carbonsäure, 7-Chlor-6-fluor-1-(2,4-difluorphenyl)-1,4-dihydro-5-methyl-4-oxo-1,8-naphthyridin-3-carbonsäure, 6,7-Difluor-1,4-dihydro-1-(3-oxetanyl)-4-oxo-3-chinolincarbonsäure, 6,7,8-Trifluor-1,4-dihydro-1-(3-oxetanyl)-4-oxo-3-chinolincarbonsäure, 1-(Bicyclo[1.1.1]pent-1-yl)-6,7,8-trifluor-1,4-dihydro-4-oxo-3-chinolincarbonsäure, 7-Chlor-1-(1,1-dimethylpropargyl)-6-fluor-1,4-dihydro-4-oxo-1,8-naphthyridin-3-carbonsäure, 6,7,8-Trifluor-1-(2,4-difluorphenyl)-1,4-dihydro-4-oxo-3-chinolincarbonsäure, 6,7,8-Trifluor-1,4-dihydro-4-oxo-1-phenyl-3-chinolincarbonsäure, 7-Chlor-1-ethyl-6-fluor-1,4-dihydro-4-oxo-1,8-naphthyridin-3-carbonsäure, 6,7-Difluor-1,4-dihydro-4-oxo-1-vinyl-3-chinolincarbonsäure, 1-Cyclopropyl-5,6,7,8-tetrafluor-1,4-dihydro-4-oxo-3-chinolincarbonsäure, 5-Amino-1-cyclopropyl-6,7,8-trifluor-1,4-dihydro-4-oxo-3-chinolincarbonsäure, 1-Cyclopropyl-6,7,8-trifluor-1,4-dihydro-5-hydroxy-4-oxo-3-chinolincarbonsäure, 1-Cyclopropyl-6,7-difluor-1,4-dihydro-8-methoxy-4-oxo-3-chinolincarbonsäure, 7-Chlor-1-(2,4-difluorphenyl)-6-fluor-1,4-dihydro-4-oxo-1,8-naphythyridin-3-carbonsäureethylester, 1-Cyclopropyl-6,7-difluor-1,4-dihydro-4-oxo-8-vinyl-3-chinolincarbonsäure, 1-Cyclopropyl-8-ethinyl-6,7-difluor-1,4-dihydro-4-oxo-3-chinolincarbonsäure, 9,10-Difluor-3-methyl-7-oxo-2,3-dihydro-7H-pyrido[1,2,3-d,e][1,3,4]benzoxadiazin-6-carbonsäure, 8-Amino-9,10-difluor-3-methyl-7-oxo-2,3-dihydro-7H-pyrido[1,2,3-d,e][1,3,4]benzoxadiazin-6-carbonsäure, 7,8-Difluor-5-oxo-9,1-[(N-methylimino)methano]-5H-thiazolo[3,2-a]-chinolin-4-carbonsäure, 7,8-Difluor-5-oxo-9,1-[(N-ethylimino)methano]-5H-thiazolo[3,2-a]chinolin-4-carbonsäure,7,8-Difluor-5-oxo-9,1-(epoxymethano)-5H-thiazolo[3,2-a]chinolin-4-carbonsäure, 7,8-Difluor-5-oxo-9,1-(epithiomethano)-5H-thiazolo[3,2-a]-chinolin-4-carbonsäure, 7,8-Difluor-1-methyl-5-oxo-5H-thiazolo[3,2-a]-chinolin-4-carbonsäure, 8-Brom-6,7-difluor-1-(cis-2-fluorcyclopropyl)-1,4-dihydro-4-oxo-3-chinolincarbonsäure,8-Chlor-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-3-chinolincarbonsäure, 6,7,8-Trifluor-1-(cis-2-fluorcyclopropyl)-1,4-dihydro-4-oxo-3-chinolincarbonsäure, 5,6,7,8-Tetrafluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-8-methyl-4-oxo-3-chinolincarbonsäure, 8-Ethinyl-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-8-trifluormethyl-3-chinolincarbonsäure, 6,7-Difluor-1-(cis-2-fluorcyclopropyl)-8-difluormethoxy-1,4-dihydro-4-oxo-3-chinolincarbonsäure, 6,7-Difluor-1-(cis-2-fluorcyclopropyl)-1,4-dihydro-8-methoxy-4-oxo-3-chinolincarbonsäure, 6,7-Difluor-1-(cis-2-fluorcyclopropyl)-1,4-dihydro-5-methyl-4-oxo-3-chinolincarbonsäure, 5-Amino-6,7,8-trifluor-1-(cis-2-fluorcyclopropyl)-1,4-dihydro-4-oxo-3-chinolincarbonsäure, 8-Brom-6,7-difluor-1-[(1R,2S)-2-fluorcyclopropyl]-1,4-dihydro-4-oxo-3-chinolincarbonsäure, 8-Chlor-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-3-chinolincarbonsäure, 6,7,8-Trifluor-1-[(1R,2S)-2-fluorcyclopropyl]-1,4-dihydro-4-oxo-3-chinolincarbonsäure, 5,6,7,8-Tetrafluor-1-((1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-8-methyl-4-oxo-3-chinolincarbonsäure, 8-Ethinyl-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-8-trifluormethyl-3-chinolincarbonsäure, 6,7-Difluor-1-[(1R,2S)-2-fluorcyclopropyl]-8-difluormethoxy-1,4-dihydro-4-oxo-3-chinolincarbonsäure, 6,7-Difluor-1-[(1R,2S)-2-fluorcyclopropyl]-1,4-dihydro-8-methoxy-4-oxo-3-chinolincarbonsäure, 6,7-Difluor-1-[(1R,2S)-2-fluorcyclopropyl]-1,4-dihydro-5-methyl-4-oxo-3-chinolincarbonsäure, 5-Amino-6,7,8-Trifluor-1-[(1R,2S)-2-fluorcyclopropyl]-1,4-dihydro-4-oxo-3-chinolincarbonsäure.

Die als Ausgangsverbindungen benötigten tricyclischen Amine der Formel (III) sind neu. Sie können nach den im Schema 1 wiedergegebenen Methoden hergestellt werden: Ein cyclisches Dien (1) wird im Sinne einer Diels-Alder-Reaktion mit einem Imid (2) zum Addukt (3) umgesetzt. Die Verbindungen (3) können als Nitrile (G = CN) oder Ester (G = COOC₂H₅) über Amidzwischenstufen (4) zu Aminen (5) abgebaut werden, die sich beispielsweise mit komplexen Hydriden zu den tricyclischen Diaminen (6) hydrieren lassen. Falls in (6) der Rest R für Benzyl kann dieser hydrogenolytisch unter gleichzeitiger Hydrierung der Doppelbindung unter Bildung des gesättigten tricyclischen Diamins (7) abgespalten werden. Die Verbindungen (3) mit Substituenten wie beispielsweise G = OCH₃, OH, CN, COOC₂H₅ oder N(CH₃)₂ können auch direkt mit komplexen Hydriden zu den Verbindungen (8) reduziert und diese anschließend zu den gesättigten tricyclischen Diaminen (9) hydriert werden. Selektiv acyclierte Diamine lassen sich beispielsweise über eine tert.-Butoxycarbonylierung der Verbindung (6) (R = Benzyl) und anschließende Hydrierung zu (10) herstellen. Monomethylierte Diamine (11) oder (12) lassen sich beispielsweise über eine Acylierung von (5) mit einem Chlorameisensäureester und nachfolgende Reduktion zu (11) beziehungsweise zu (12) herstellen. Die in dem Formelschema aufgeführten Verbindungen (6) bis (12) entsprechen der allgemeinen Struktur (III), wenn R = H bedeutet.

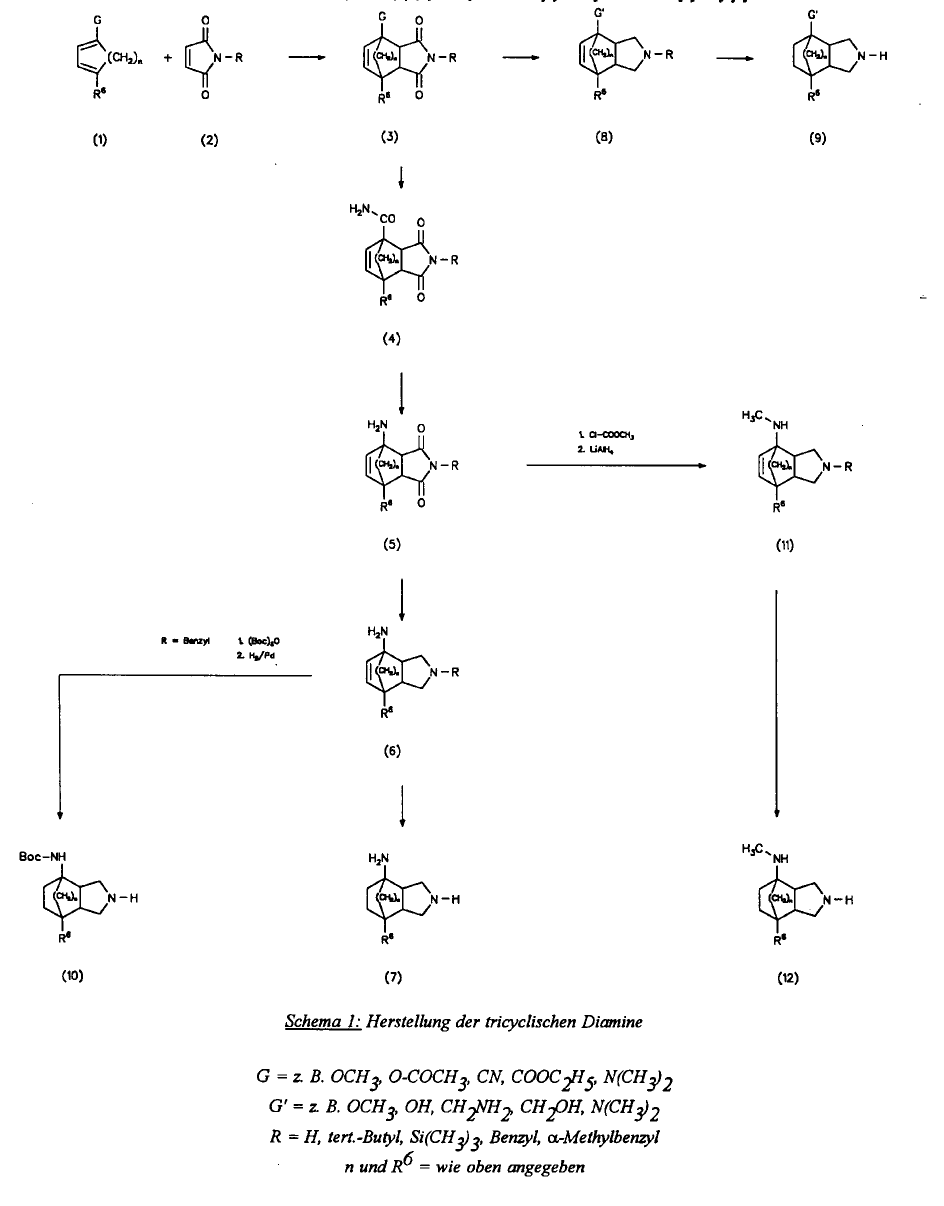

Als Beispiele für tricyclische Amine der Formel (III) seien genannt:
4-Azatricyclo[5.2.1.0^{2,6}]dec-8-en-1-ylamin, 4-Azatricyclo[5.2.2.0^{2,6}]undec-8-en-1-ylamin, 4-Azatricyclo[5.2.1.0^{2,6}]dec-8-en-1-ylamin-N-carbonsäure-tert-butylester, 4-Azatricyclo[5.2.2.0^{2,6}]undec-8-en-1-ylamin-N-carbonsäure-tert.-butylester, (4-Azatricyclo[5.2.1.0^{2,6}]dec-8-en-1-yl)-methyl-amin, (4-Azatricyclo[5.2.2.0^{2,6}]undec-8-en-1-yl)-methyl-amin, (4-Azatricyclo[5.2.1.0^{2,6}]dec-8-en-1-yl)dimethyl-amin, (4-Azatricyclo[5.2.2.0^{2,6}]undec-8-en-1-yl)-dimethyl-amin, 7-Methyl-4-azatricyclo[5.2.1.0^{2,6}]dec-8-en-1-yl-amin,7-Methyl-4-azatricyclo[5.2.2.0^{2,6}]undec-8-en-1-yl-amin, Methyl-(7-methyl-4-azatricyclo[5.2.1.0^{2,6}]dec-8-en-1-yl)-amin, Methyl-(7-methyl-4-azatricyclo[5.2.2.0^{2,6}]undec-8-en-1-yl)-amin, Dimethyl-(7-methyl-4-azatricyclo[5.2.1.0^{2,6}]dec-8-en-1-yl)-amin, Dimethyl-(7-methyl-4-azatricyclo[5.2.2.0^{2,6}]undec-8-en-1-yl)amin, (4-Azatricyclo[5.2.1.0^{2,6}]dec-8-en-1-yl-methyl)amin, (4-Azatricyclo[5.2.2.0^{2,6}]undec-8-en-1-ylmethyl)amin, 4-Azatricyclo[5.2.1.0^{2,6}]dec-8-en-1-ol, 4-Azatricyclo[5.2.2.0^{2,6}]undec-8-en-1-ol, (4-Azatricyclo[5.2.1.0^{2,6}]dec-8-en-1-yl)-methanol, (4-Azatricyclo[5.2.2.0^{2,6}]undec-8-en-1-yl)-methanol, 4-Azatricyclo[5.2.1.0^{2,6}]dec-1-ylamin, 4-Azatricyclo[5.2.2.0^{2,6}]undec-1-ylamin, 4-Azatricyclo[5.2.1.0^{2,6}]dec-1-ylamin-N-carbonsäure-tert.-butyester, 4-Azatricyclo[5.2.2.0^{2,6}]undec-1-ylamin-N-carbonsäure-tert.-butylester, (4-Azatricyclo[5.2.1.0^{2,6}]dec-1-yl)-methyl-amin, (4-Azatricyclo[5.2.2.0^{2,6}]undec-1-yl)-methyl-amin, (4-Azatricyclo[5.2.1.0^{2,6}]dec-1-yl)-dimethyl-amin, (4-Azatricyclo[5.2.2.0^{2,6}]undec-1-yl)-dimethyl-amin, 7-Methyl-4-azatricyclo[5.2.1.0^{2,6}]dec-1-yl-amin, 7-Methyl-4-azatricyclo[5.2.2.0^{2,6}]undec-1-yl-amin, Methyl-(7-methyl-4-azatricyclo[5.2.1.0^{2,6}]dec-1-yl)-amin, Methyl-(7-methyl-4-azatricyclo[5.2.2.0^{2,6}]undec-1-yl)-amin, Dimethyl-(7-methyl-4-azatricyclo[5.2.1.0^{2,6}]dec-1-yl)-amin, Dimethyl-(7-methyl-4-azatricyclo[5.2.2.0^{2,6}]undec-1-yl)-amin, (4-Azatricyclo[5.2.1.0^{2,6}]dec-1-ylmethyl)amin, (4-Azatricyclo[5.2.2.0^{2,6}]undec-1-yl-methyl)amin,4-Azatricyclo[5.2.1.0^{2,6}]dec-1-ol,4-Azatricyclo[5.2.2.0^{2,6}]undec-1-ol,(4-Azatricyclo[5.2.1.0^{2,6}]dec-1-yl)-methanol, (4-Azatricyclo[5.2.2.0^{2,6}]undec-1-yl)-methanol.

Die als Ausgangsverbindungen verwendeten enantiomerenreinen Verbindungen der Formel (III) können nach folgenden Verfahren hergestellt werden:
1. Die racemischen tricyclischen Amine (III) können mit enantiomerenreinen Säuren, zum Beispiel Carbonsäuren oder Sulfonsäuren wie N-Acetyl-L-glutaminsäure, N-Benzoyl-L-alanin, 3-Brom-campher-9-sulfonsäure, Campher-3-carbonsäure, cis-Camphersäure, Campher-10-sulfonsäure, O,O'-Dibenzoyl-weinsäure, D- oder L-Weinsäure, Mandelsäure, α-Methoxy-phenylessigsäure, 1-Phenyl-ethansulfonsäure, α-Phenyl-bernsteinsäure zu einem Gemisch der diastereomeren Salze umgesetzt werden, die sich durch fraktionierte Kristallisation zu den diastereomerenreinen Salzen trennen lassen (siehe P. Newman, Opical Resolution Procedures for Chemical Compounds, Volume 1). Durch Behandlung dieser Salze mit Alkali- oder Erdalkalihydroxyden lassen sich die enantiomerenreinen Amine freisetzen.
2. In ähnlicher Weise wie unter 1. beschrieben läßt sich eine Racematspaltung der basischen Zwischenstufen, die bei der Herstellung der raceinischen bicyclischen Amine auftreten, mit den oben aufgeführten enantiomerenreinen Säuren durchführen. Beispiele für derartige basische Zwischenstufen sind Verbindungen der Struktur (5) in Schema 1.
3. Sowohl die racemischen Amine (III) als auch die Zwischenstufen bei ihrer Herstellung, wie zum Beispiel Verbindungen der Strukturen (3) bis (5) in Schema 1, können, gegebenenfalls nach Acylierung, über chirale Trägermaterialien chromatographisch getrennt werden (siehe zum Beispiel G. Blaschke, Angew. Chem. **92**, 14 [1980]).
4. Sowohl die racemischen Amine (III) als auch basische Zwischenstufen, wie zum Beispiel Verbindungen der Struktur (5) in Schema 1, können durch chemische Verknüpfung mit chiralen Acylresten in Diastereomerengemische überführt werden, die sich durch Destillation, Kristallisation oder Chromatographie in die diastereomerenreinen Acylderivate trennen lassen, aus denen sich durch Verseifung die enantiomerenreinen Amine isolieren lassen. Beispiele für Reagentien zur Verknüpfung mit chiralen Acylresten sind: α-Methoxy-α-trifluormethyl-phenylacetylchlorid, Menthylisocyanat, D- oder L-α-Phenyl-ethyl-isocyanat, Chlorameisensäure-menthylester, Campher-10-sulfonsäurechlorid.
5. Im Verlauf der Synthese der tricyclischen Amine (III) können statt achiraler auch chirale Schutzgruppen eingeführt werden. Man gelangt auf diese Weise zu Diastereomeren, die sich trennen lassen. Zum Beispiel kann bei der Synthese der Verbindung (3) in Schema 1 der Benzylrest durch den R- oder S-konfigurierten α-Phenylethylrest ersetzt werden.

Die Umsetzung von (II) mit (III), bei der die Verbindungen (III) auch in Form ihrer Salze, wie zum Beispiel der Hydrochloride, eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 160°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 5 Mol der Verbindung (III) ein.

Freie Aminogruppen können während der Umsetzung durch eine geeignete Aminoschutzgruppe, wie zum Beispiel durch den tert.-Butoxycarbonylrest oder eine Azomethin-Schutzgruppe, geschützt und nach Beendigung der Reaktion wieder freigesetzt werden.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure,p-Toluolsulfonsäure oder sauren Ionenaustauschern, bei Temperaturen von etwa 20 bis 180°C, vorzugsweise etwa 60 bis 120°C, umgesetzt. Das entstehende Reaktionswasser kann auch durch azeotrope Destillation mit Chloroform, Tetrachlormethan oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten Ester, wie zum Beispiel (5-Methyl-2-oxo-1,3-dioxol-4-yl-methyl)-ester, werden durch Umsetzung eines Alkalisalzes der zugrundliegenden Carbonsäure, die gegebenenfalls am N-Atom durch eine Schutzgruppe geschützt sein kann, mit 4-Brommethyl- oder 4-Chlormethyl-5-methyl-1,3-dioxol-2-on in einem Lösungsmittel wie Dimethylformamid,Dimethylacetamid,N-Methyl-pyrrolidon,Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen in überschüssiger wäßriger Säure und Ausfällen des Salzes mit einem mit Wasser mischbaren Lösungsmittel wie Methanol, Ethanol, Aceton oder Acetonitril. Man kann auch äquivalente Menge Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, 2-Hydroxyglutarsäure, Methansulfonsäure, 4-Toluolsulfonsäure, Galacturonsäure, Glucuronsäure, 5-Oxotetrahydrofuran-2-carbonsäure, Embonsäure, Glutaminsäure oder Asparaginsäure zu verstehen.

Die Alkali- und Erdalkalisalze der erfindungsgemäßen Carbonsäuren werden beispielsweise durch Lösen des Betains in überschüssiger Alkali- oder Erdalkalilauge, Filtration vom ungelösten Betain und Eindampfen bis zur Trockne erhalten. Pharmazeutisch geeignet sind Natrium-, Kalium- und Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die nachfolgend aufgeführten sowie die in den Tabellen 1 bis 8 aufgeführten Wirkstoffe hergestellt werden, die sowohl als Racemate oder als enantiomerenreine Verbindungen oder gegebenenfalls auch als Diastereomerengemische oder als diastereomerenreine Verbindungen vorliegen können:
8-(1-Amino-4-azatricyclo[5.2.1.0^{2,6}]dec-8-en-4-yl)-7-fluor-5-oxo-9,1-(epoxymethano)-5H-thiazolo[3,2-a]chinolin-4-carbonsäure, 8-(1-Methylamino-4-azatricyclo[5.2.1.0^{2,6}]dec-8-en-4-yl)-7-fluor-5-oxo-9,1-(epoxymethano)-5H-thiazolo[3,2-a]chinolin-4-carbonsäure, 8-(1-Amino-7-methyl-4-azatricyclo[5.2.1.0^{2,6}]dec-8-en-4-yl)-7-fluor-5-oxo-9,1-(epoxymethano)-5H-thiazolo[3,2-a]chinolin-4-carbonsäure, 8-(1-Amino-4-azatricyclo[5.2.1.0^{2,6}]dec-4-yl)-7-fluor-5-oxo-9,1-(epoxymethano)-5H-thiazolo[3,2-a]chinolin-4-carbonsäure, 8-(1-Methylamino-4-azatricyclo[5.2.1.0^{2,6}]dec-4-yl)-7-fluor-5-oxo-9,1-(epoxymethano)-5H-thiazolo[3,2-a]chinolin-4-carbonsäure, 8-(1-Amino-7-methyl-4-azatricyclo[5.2.1.0^{2,6}]dec-4-yl)-7-fluor-5-oxo-9,1-(epoxymethano)-5H-thiazolo[3,2-a]chinolin-4-carbonsäure, 8-(1-Amino-4-azatricyclo[5.2.1.0^{2,6}]dec-8-en-4-yl)-7-fluor-5-oxo-9,1-[(N-methyl-imino)methano]-5H-thiazolo[3,2-a]chinolin-4-carbonsäure, 8-(1-Methylamino-4-azatricyclo[5.2.1.0^{2,6}]dec-8-en-4-yl)-7-fluor-5-oxo-9,1-[(N-methyl-imino)methano]-5H-thiazolo[3,2-a]chinolin-4-carbonsäure, 8-(1-Amino-7-methyl-4-azatricyclo[5.2.1.0^{2,6}]dec-8-en-4-yl)-7-fluor-5-oxo-9,1-[(N-methylimino)methano]-5H-thiazolo[3,2-a]chinolin-4-carbonsäure, 8-(1-Amino-4-azatricyclo[5.2.1.0^{2,6}]dec-4-yl)-7-fluor-5-oxo-9,1-[(N-methyl-imino)methano]-5H-thiazolo[3,2-a]chinolin-4-carbonsäure, 8-(1-Methylamino-4-azatricyclo[5.2.1.0^{2,6}]dec-4-yl)-7-fluor-5-oxo-9,1-[(N-methyl-imino)methano]-5H-thiazolo[3,2-a]chinolin-4-carbonsäure, 8-(1-Amino-7-methyl-4-azatricyclo[5.2.1.0^{2,6}]dec-4-yl)-7-fluor-5-oxo-9,1-[(N-methylimino)methano]-5H-thiazolo[3,2-a]chinolin-4-carbonsäure, 8-Amino-10-(1-amino-4-azatricyclo[5.2.1.0^{2,6}]decan-4-yl)-9-fluor-3-methyl-7-oxo-2,3-dihydro-7H-pyrido[1,2,3-d,e][1,3,4]benzoxadiazin-6-carbonsäure, 10-(1-Amino-4-azatricyclo[5.2.1.0^{2,6}]decan-4-yl)-9-fluor-3-methyl-7-oxo-2,3-dihydro-7H-pyrido[1,2,3-d,e][1,3,4]benzoxadiazin-6-carbonsäure, 10-(1-Amino-7-methyl-4-azatricyclo[5.2.1.0^{2,6}]decan-4-yl)-9-fluor-3-methyl-7-oxo-2,3-dihydro-7H-pyrido[1,2,3-d,e][1,3,4]benzoxadiazin-6-carbonsäure, 10-(1-Methylamino-4-azatricyclo[5.2.1.0^{2,6}]decan-4-yl)-9-fluor-3-methyl-7-oxo-2,3-dihydro7H-pyrido[1,2,3-d,e][1,3,4]benzoxadiazin-6-carbonsäure, 10-(1-Amino-4-azatricyclo-[5.2.1.0^{2,6}]dec-8-en-4-yl)-9-fluor-3-methyl-7-oxo-2,3-dihydro-7H-pyrido[1,2,3-d,e]-[1,3,4]benzoxadiazin-6-carbonsäure,10-(1-Methylamino-4-azatricyclo[5.2.1.0^{2,6}]dec-8-en-4-yl)-9-fluor-3-methyl-7-oxo-2,3-dihydro-7H-pyrido[1,2,3-d,e][1,3,4]benzoxadiazin-6-carbonsäure, 10-(1-Amino-7-methyl-4-azatricyclo[5.2.1.0^{2,6}]dec-8-en-4-yl)-9-fluor-3-methyl-7-oxo-2,3-dihydro-7H-pyrido[1,2,3-d,e][1,3,4]benzoxadiazin-6-carbonsäure, 10-(1-Amino-4-azatricyclo[5.2.2.0^{2,6}]undec-8-en-4-yl)-9-fluor-3-methyl-7-oxo-2,3-dihydro-7H-pyrido[1,2,3-d,e][1,4]benzoxazin-6-carbonsäure, 10-(1-Amino-4-azatricyclo-[5.2.2.0^{2,6}]undecan-4-yl)-9-fluor-3-methyl-7-oxo-2,3-dihydro-7H-pyrido[1,2,3-d,e][1,4]benzoxazin-6-carbonsäure, 8-Amino-10-(1-amino-4-azatricyclo[5.2.1.0^{2,6}]decan-4-yl)-9-fluor-3-methyl-7-oxo-2,3-dihydro-7H-pyrido[1,2,3-d,e][1,4]benzoxazin-6-carbonsäure, 10-(1-Amino-4-azatricyclo[5.2.1.0^{2,6}]decan-4-yl)-9-fluor-3-methyl-7-oxo-2,3-dihydro-7H-pyrido[1,2,3-d,e][1,4]benzoxazin-6-carbonsäure, 10-(1-Amino-7-methyl-4-azatricyclo[5.2.1.0^{2,6}]decan-4-yl)-9-fluor-3-methyl-7-oxo-2,3-dihydro-7H-pyrido[1,2,3-d,e][1,4]benzoxazin-6-carbonsäure, 10-(1-Methylamino-4-azatricyclo[5.2.1.0^{2,6}]decan-4-yl)-9-fluor-3-methyl-7-oxo-2,3-dihydro-7H-pyrido[1,2,3-d,e][1,3,4]benzoxadiazin-6-carbonsäure, 10-(1-Amino-1-azatricyclo[5.2.1.0^{2,6}]dec-8-en-4-yl)-9-fluor-3-methyl-7-oxo-2,3-dihydro-7H-pyrido[1,2,3-d,e][1,4]benzoxazin-6-carbonsäure, 10-(1-Methylamino-4-azatricyclo[5.2.1.0^{2,6}]dec-8-en-4-yl)-9-fluor-3-methyl-7-oxo-2,3-dihydro-7H-pyrido[1,2,3-d,e][1,4]benzoxazin-6-carbonsäure, 10-(1-Amino-7-methyl-4-azatricyclo[5.2.1.0^{2,6}]dec-8-en-4-yl)-9-fluor-3-methyl-7-oxo-2,3-dihydro-7H-pyrido[1,2,3-d,e][1,4]benzoxazin-6-carbonsäure, 8-(1-Aminomethyl-4-azatricyclo[5.2.1.0^{2,6}]dec-4-yl)-7-fluor-5-oxo-9,1-(epoxymethano)-5H-thiazolo[3,2-a]chinolin-4-carbonsäure, 8-(1-Aminomethyl-4-azatricyclo[5.2.2.0^{2,6}]undec-4-yl)-7-fluor-5-oxo-9,1-(epoxymethano)-5H-thiazolo[3,2-a]chinolin-4-carbonsäure, 8-(1-Aminomethyl-4-azatricyclo[5.2.1.0^{2,6}]dec-8-en-4-yl)-7-fluor-5-oxo-9,1-(epoxymethano)-5H-thiazolo[3,2-a]chinolin-4-carbonsäure, 8-(1-Aminomethyl-4-azatricyclo[5.2.2.0^{2,6}]-undec-8-en-4-yl)-7-fluor-5-oxo-9,1-(epoxymethano)-5H-thiazolo[3,2-a]chinolin-4-carbonsäure, 8-(1-Aminomethyl-4-azatricyclo[5.2.1.0^{2,6}]dec-4-yl)-7-fluor-5-oxo-9,1-[(N-methyl-imino)methano]-5H-thiazolo[3,2-a]chinolin-4-carbonsäure, 8-(1-Aminomethyl-4-azatricyclo[5.2.2.0^{2,6}]undec-4-yl)-7-fluor-5-oxo-9,1-[(N-methyl-imino)methano]-5H-thiazolo[3,2-a]chinolin-4-carbonsäure, 8-(1-Aminomethyl-4-azatricyclo[5.2.1.0^{2,6}]dec-8-en-4-yl)-7-fluor-5-oxo-9,1-[(N-methyl-imino)methano]-5H-thiazolo[3,2-a]chinolin-4-carbonsäure, 8-(1-Aminomethyl-4-azatricyclo[5.2.2.0^{2,6}]-undec-8-en-4-yl)-7-fluor-5-oxo-9,1-[(N-methyl-imino)methano]-5H-thiazolo[3,2-a]chinolin-4-carbonsäure, 10-(1-Aminomethyl-4-azatricyclo[5.2.1.0^{2,6}]dec-4-yl)-9-fluor-3-methyl-7-oxo-2,3-dihydro-7H-pyrido[1,2,3-d,e][1,3,4]benzoxadiazin-6-carbonsäure, 10-(1-Aminomethyl-4-azatricyclo[5.2.2.0^{2,6}]undec-4-yl)-9-fluor-3-methyl-7-oxo-2,3-dihydro-7H-pyrido[1,2,3-d,e][1,3,4]benzoxadiazin-6-carbonsäure, 10-(1-Aminomethyl-4-azatricyclo[5.2.1.0^{2,6}]dec-8-en-4-yl)-9-fluor-3-methyl-7-oxo-2,3-dihydro-7H-pyrido[1,2,3-d,e][1,3,4]benzoxadiazin-6-carbonsäure, 10-(1-Aminomethyl-4-azatricyclo[5.2.2.0^{2,6}]undec-8-en-4-yl)-9-fluor-3-methyl-7-oxo-2,3-dihydro-7H-pyrido[1,2,3-d,e][1,3,4]benzoxadiazin-6-carbonsäure.

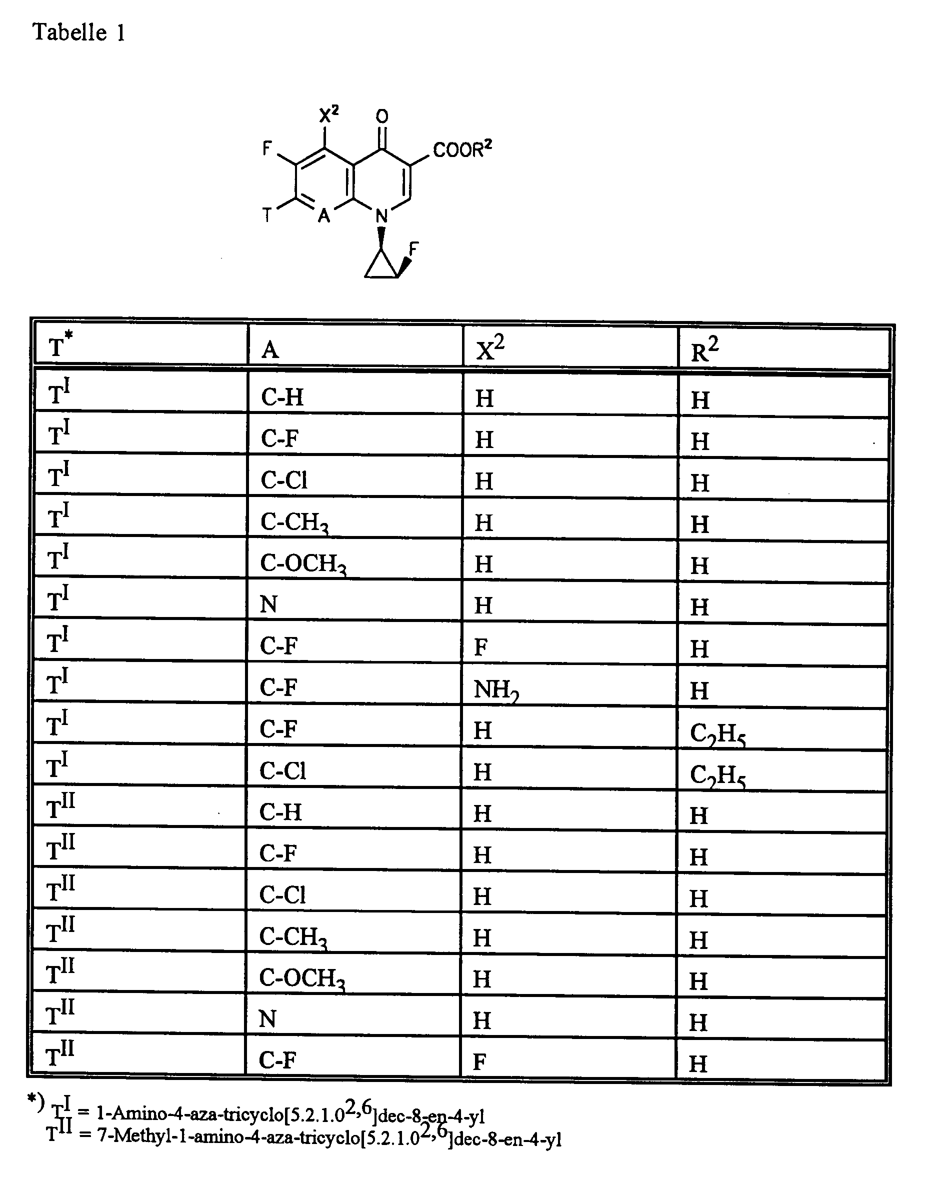

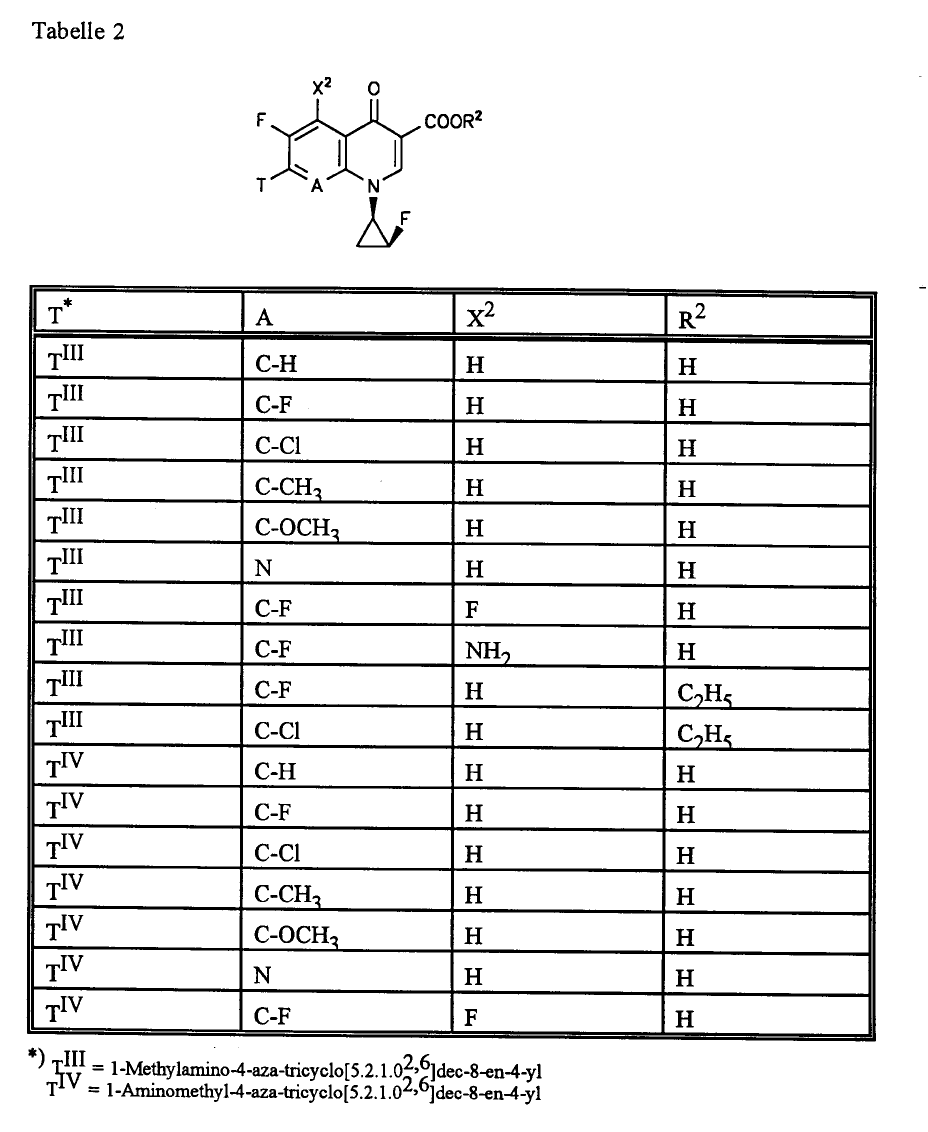

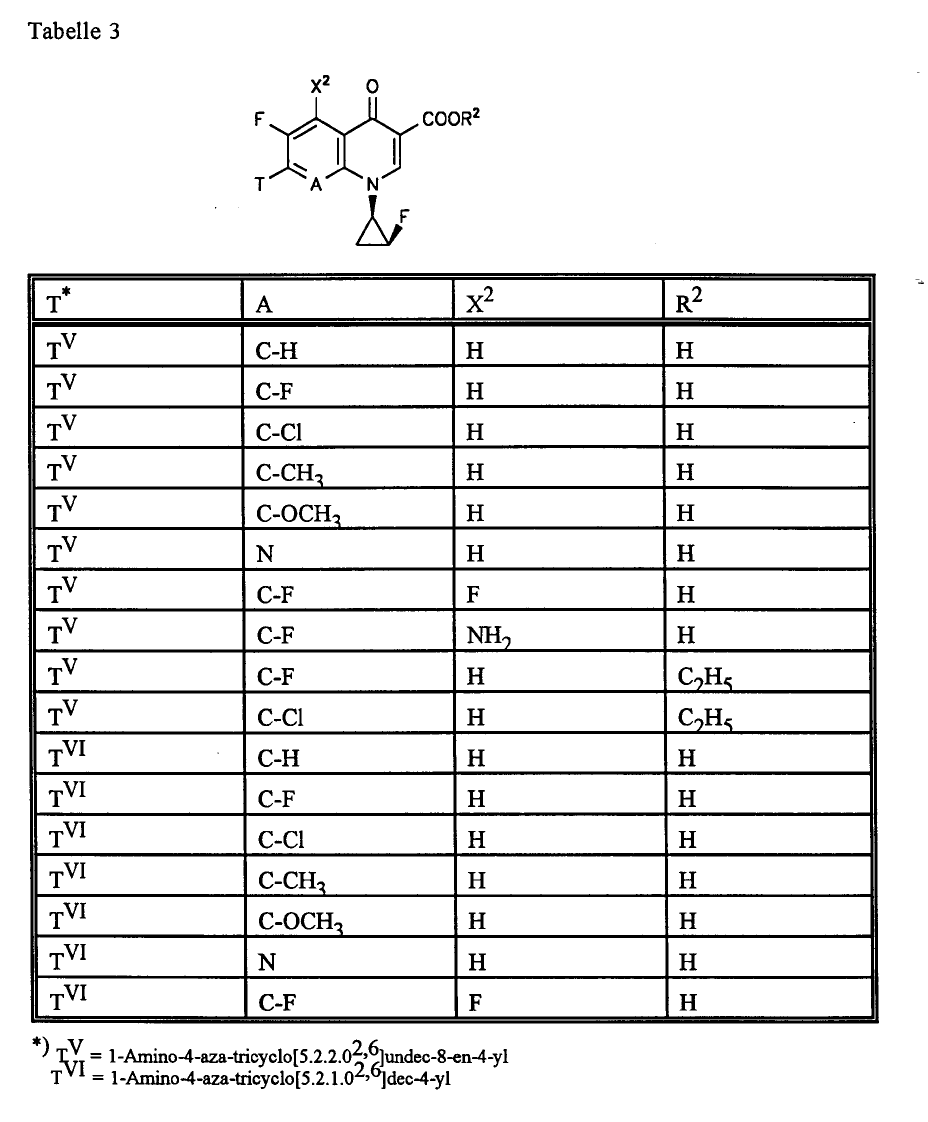

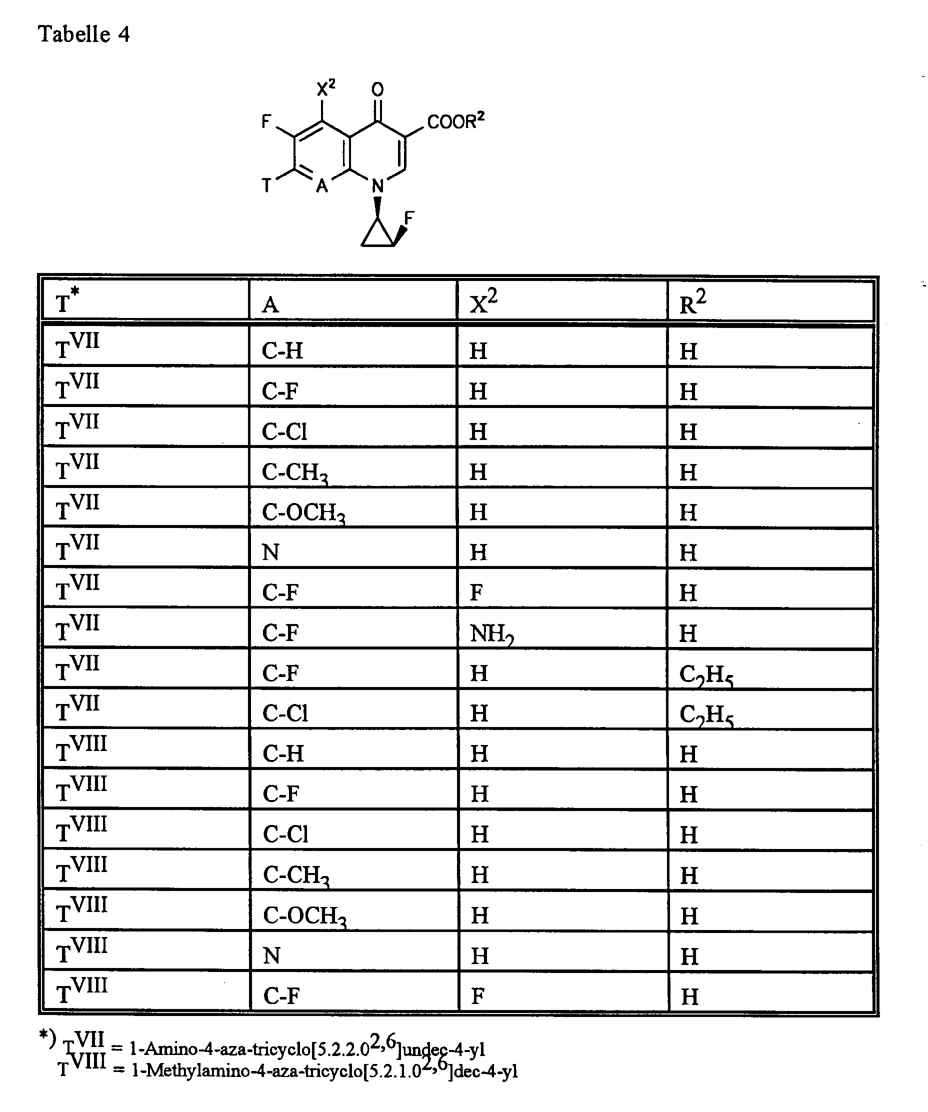

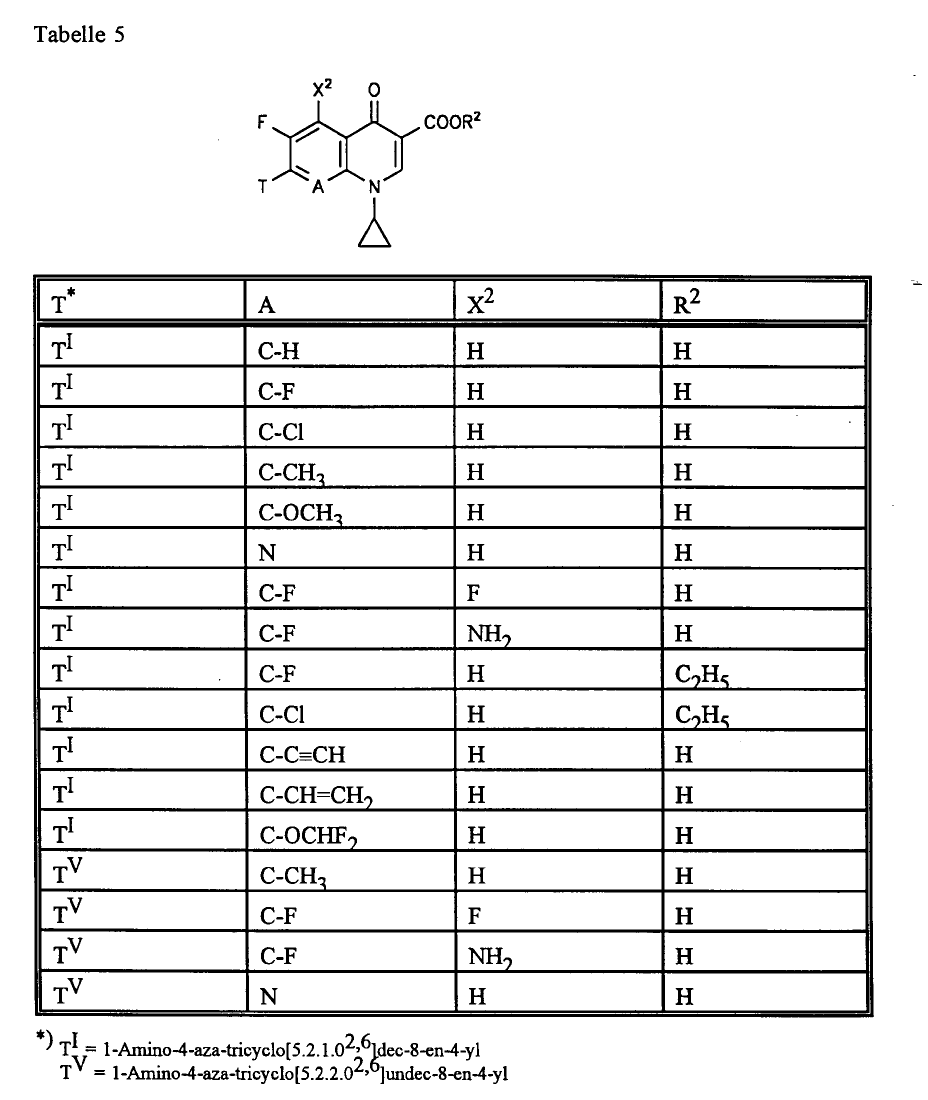

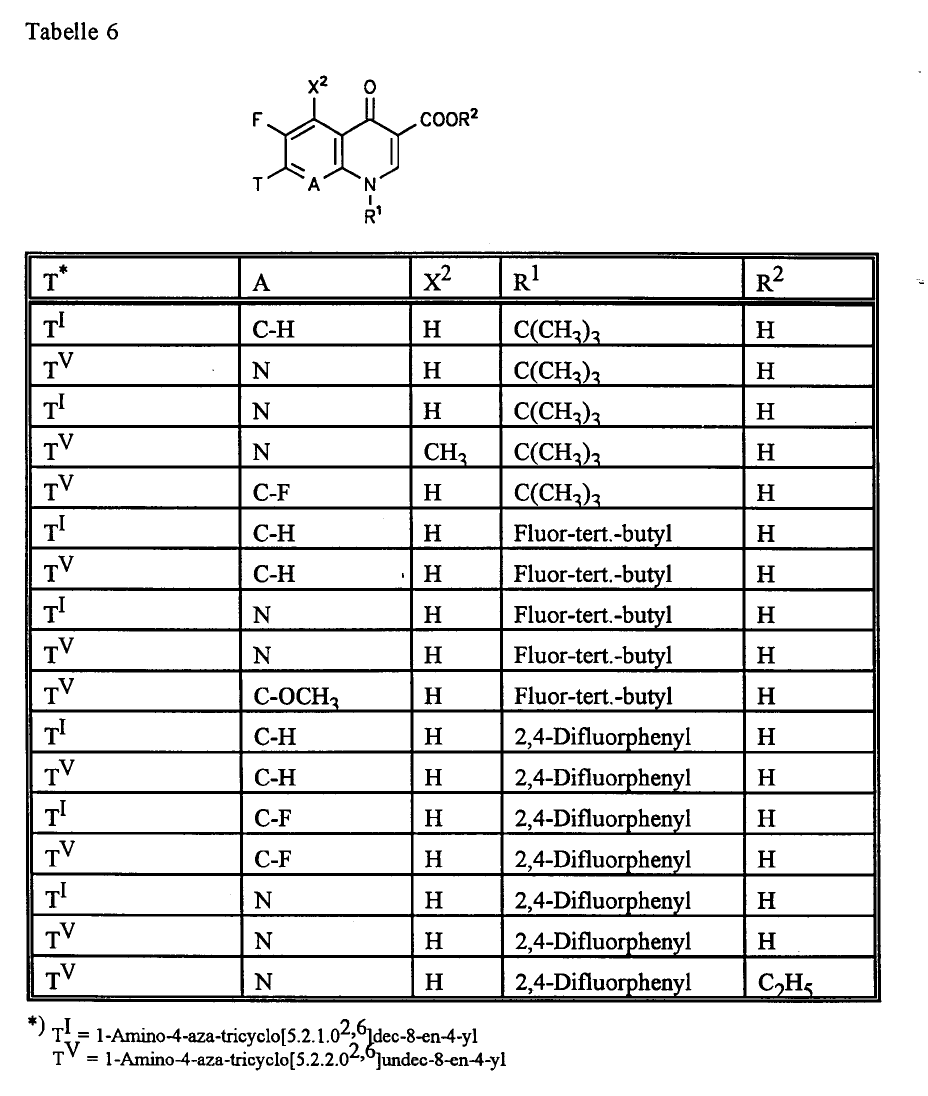

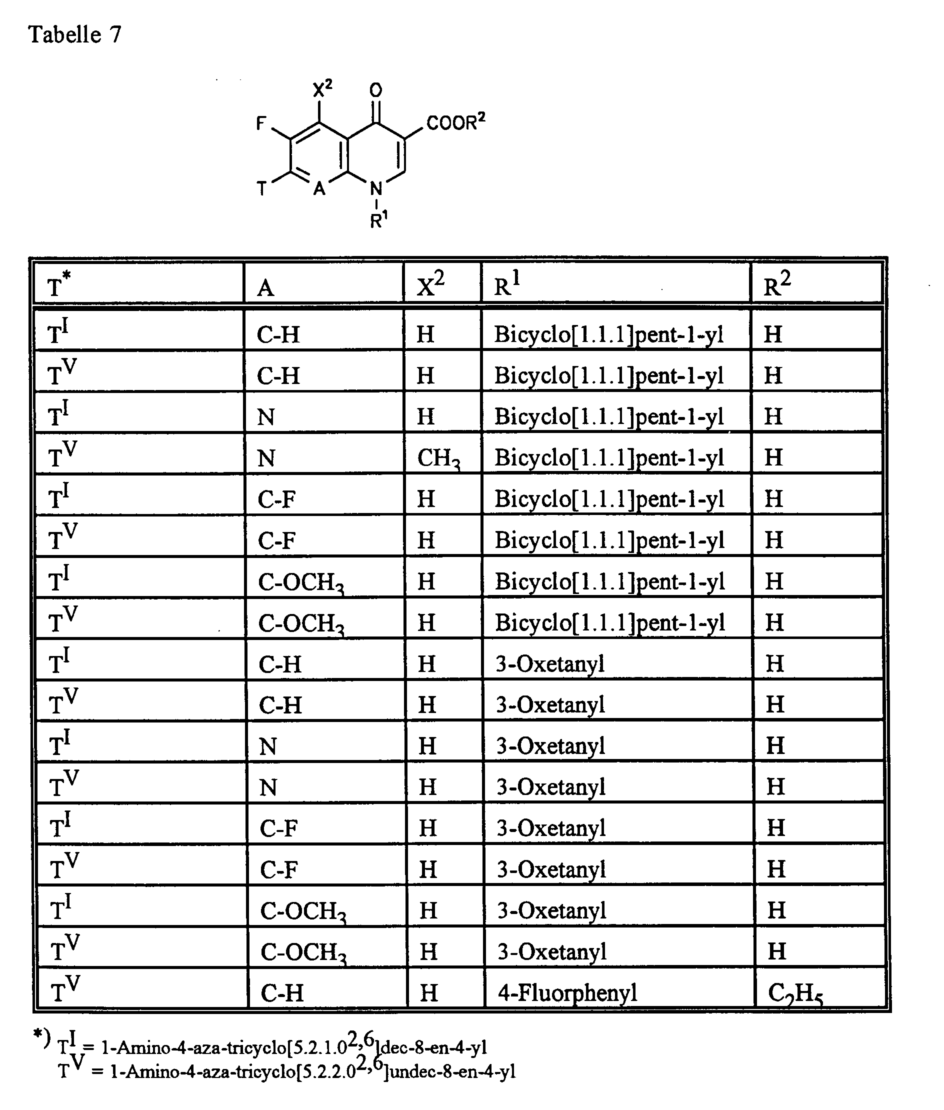

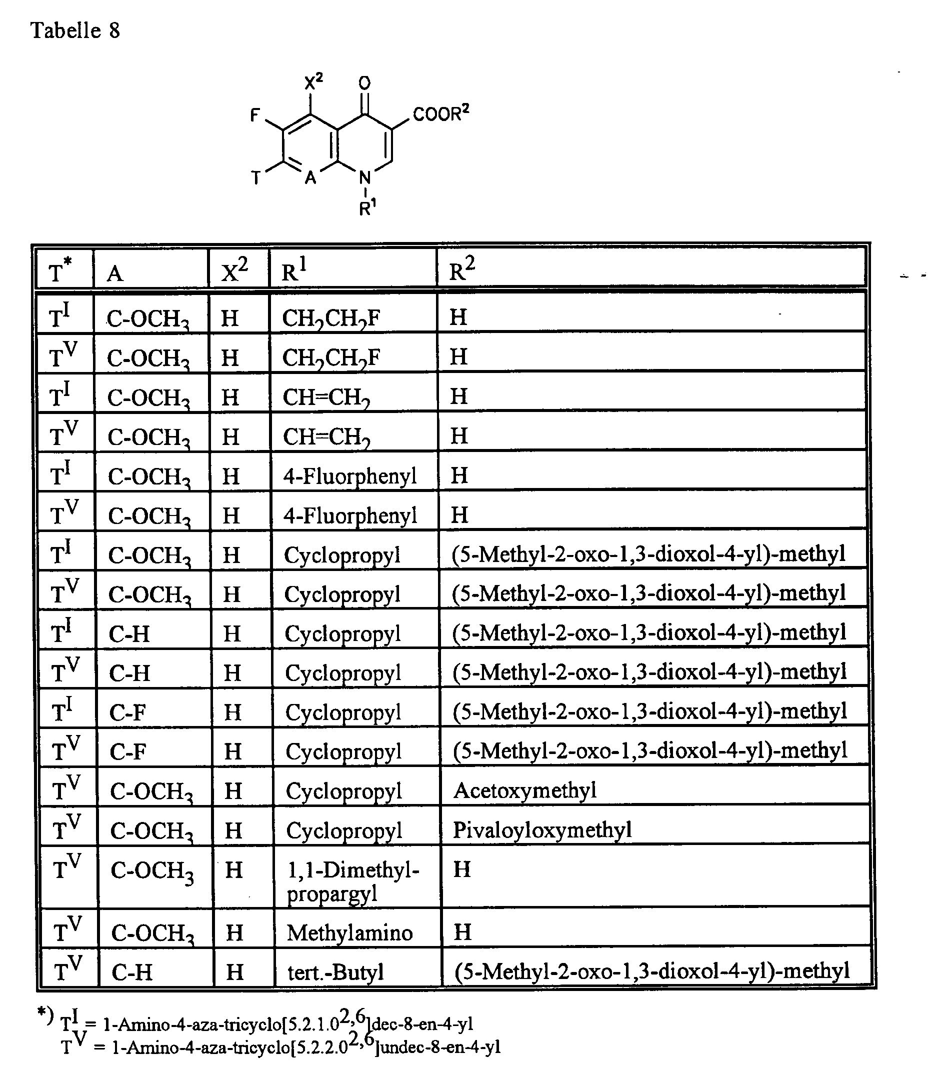

Die erfindungsgemäßen Verbindungen wirken stark antibiotisch und zeigen bei geringer Toxizität ein breites antibakterielles Spektrum gegenüber grampositiven und gramnegativen Keimen, vor allem auch gegnüber solchen, die gegen verschiedene Antibiotika, wie zum Beispiel Penicilline, Cephalosporine, Aminoglykoside, Sulfonamide, Tetracycline sowie gegen handelsübliche Chinoline, resistent sind.

Diese wertvollen Eigenschaften ermöglichen ihre Verwendung als chemotherapeutische Wirkstoffe in der Medizin sowie als Stoffe zur Konservierung von anorganischen und organischen Materialien, zum Beispiel von Polymeren, Schmiermitteln, Farben, Fasern, Leder, Papier und Holz, von Lebensmitteln und von Wasser.

Die erfindungsgemäßen Verbindungen sind gegen ein sehr breites Sprektrum von Mikroorganismen wirksam. Mit ihrer Hilfe können gramnegative und grampostive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch eine verstärkte Wirkung auf ruhende Keime aus. Bei ruhenden Bakterien, also Bakterien, die kein nachweisbares Wachstum zeigen, wirken die Verbindungen stark bakterizid. Dies bezieht sich nicht nur auf die einzusetzende Menge, sondern auch auf die Geschwindigkeit der Abtötung. Solche Ergebnisse konnten bei grampositiven und gramnegativen Bakterien, insbesondere bei Staphylococcus aureus, Pseudomonas aeruginosa, Enterococcus faecalis und Escherichia coli beobachtet werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien. Sie sind daher besonders gut in der Human- und Tiermedizin zur Prophylaxe und Chemotherapie von lokalen und systemischen Infektionen geeignet, die durch diese Erreger hervorgerufen werden.

Die Verbindungen eignen sich ferner besonders gut zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit β-Lactamderivaten wie zum Beispiel Cephalosporinen oder Penemen über kovalente Bindungen zu sogenannten Dual-Action-Derivaten verknüpft werden.

In den folgenden Tabellen 1 und 2 sind die minimalen Hemmkonzentrationen als Maß für die antibakterielle Wirksamkeit und die ID₅₀-Werte als Maß für das gentoxische Potential einer Substanz sowohl für erfindungsgemäße Verbindungen als auch für Referenzverbindungen aus dem Stand der Technik (EP 520 240) aufgeführt.

Die minimalen Hemmkonzentrationen (MHK) wurden durch ein Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Mutipoint-Inokulator (Denley) beimpft. Zum Beimpfen wurden Übernachtkulturen der Erreger verwandt, die zuvor so verdünnt wurden, daß jeder Impfpunkt circa 10⁴ koloniebildende Partikel enthielt. Die beimpften Agarplatten wurden bei 37°C bebrütet und das Keimwachstum nach circa 20 Stunden abgelesen. Der MHK-Wert (µg/ml) gibt die niedrigste Wirkstoffkonzentration an, bei der mit bloßem Auge kein Wachstum zu erkennen war.

Die erfindungsgemäßen Verbindungen zeichnen sich inbesondere dadurch aus, daß sie im Vergleich zu den Verbindungen nach dem Stand der Technik geringere Interaktionen mit Säugetier-DNA aufweisen. Unter ID₅₀ versteht man die Konzentration eines Stoffes, bei der die DNA-Synthese in Zellen aus Ovarien des chinesischen Hamsters (CHO-KI) um 50 % gehemmt wird. Dieser Wert wird nach Inkubation der entsprechenden Substanzen in abfallenden Verdünnungsstufen über definierte Zeiträume bestimmt. Dazu wird mittels fluorophotometrischer Methoden die DNA-Synthese in CHO-KI-Zellen im Vergleich zu Kontrollen ermittelt.

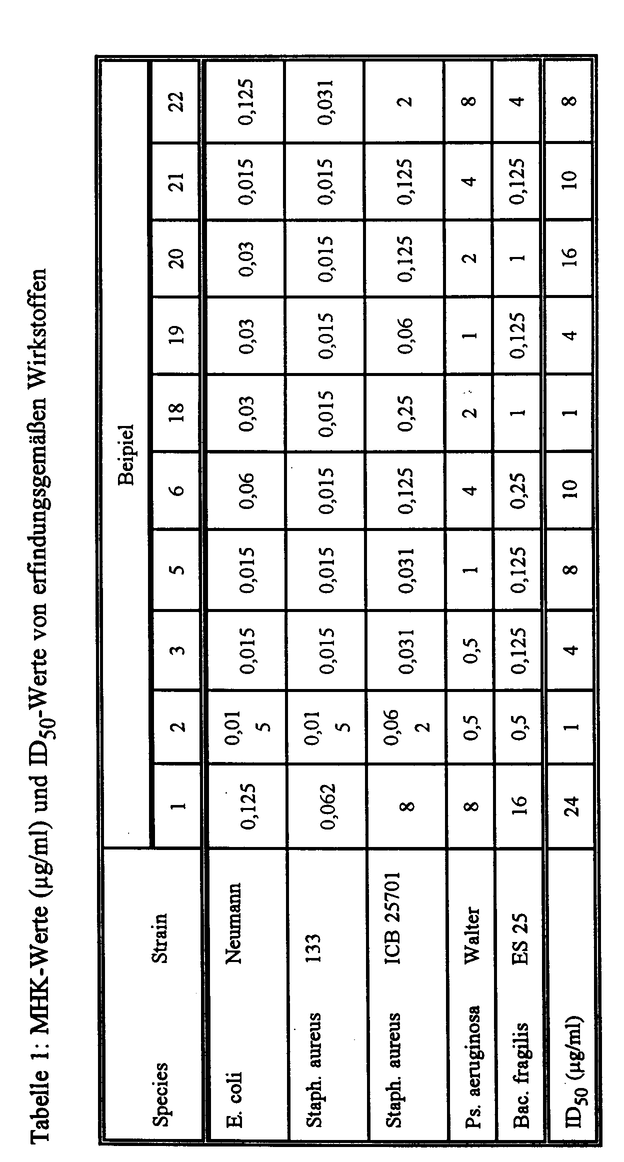

**Tabelle 2**

| MHK-Werte (µg/ml) und ID₅₀-Werte von Wirkstoffen aus dem Stand der Technik | | | | | |
|---|---|---|---|---|---|
| Species | Strain | Beispiele aus EP 520 240 | | | |
| | | 35 Ref. 1 | Ref. 2 | 36 7 Ref. 3 Ref. 4 | |
| E. coli | Neumann | 0,015 | 0,015 | 0,015 | 0,03 |
| Staph. aureus | 133 | 0,015 | 0,015 | 0,015 | 0,015 |
| Staph. aureus. | ICB 25701 | 0,06 | 0,015 | 0,015 | - |
| Ps. aeruginosa | Walter | 0,5 | 1 | 0,5 | 1 |
| Bac. fragilis | ES 25 | 0,5 | 0,25 | 0,125 | 0,5 |
| ID₅₀ (µg/ml) | | 0,015 | 0,1 | 0,1 | 0,1 |
| Ref. 1: 7-(4-Amino-7-methyl-1,3,3a,4,7,7a-hexahydro-isoindol-2-yl)-1-cyclopropyl-6,8-difluor-1,4-dihydro-4-oxo-3-chinolincarbonsäure, Ref. 2: 7-(4-Amino-7-methyl-1,3,3a,4,7,7a-hexahydro-isoindol-2-yl)-1-cyclopropyl-6-fluor-1,4-dihydro-8-methoxy-4-oxo-3-chinolincarbonsäure, Ref. 3: 7-(4-Amino-7-methyl-1,3,3a,4,7,7a-hexahydro-isoindol-2-yl)-8-chlor-1-cyclopropyl-6-fluor-1,4-dihydro-4-oxo-3-chinolincarbonsäure, Ref. 4: 7-(7-Methyl-4-methylamino-1,3,3a,4,7,7a-hexahydro-isoindol-2-yl)-8-chlor-1-cyclopropyl-6-fluor-1,4-dihydro-4-oxo-3-chinolincarbonsäure, | | | | | |

### Herstellung der Zwischenprodukte

### Beispiel Z 1

### A. 1-Methoxy-4-azatricyclo[5.2.2.0^{2.6}]undec-8-en-3,5-dion

Man erhitzt 11 g (0,1 mol) 1-Methoxycyclohexa-1,4-dien mit 0,1 g Tris(triphenylphosphin)-rutheniumdichlorid und 8,8 g (0,09 mol) Maleinimid in 100 ml absolutem Chloroform über Nacht unter Rückfluß. Man engt ein und kristallisiert aus 100 ml Toluol um.
Ausbeute: 17,6 g (84,9 % der Theorie),
Schmelzpunkt: 159-161°C.

### B. 1-Methoxy-4-azatricyclo[5.2.2.0^{2.6}]undec-8-en

Zu 6 g LiAlH₄ in 200 ml absolutem Tetrahydrofuran tropft man 20,5 g (0,1 mol) 1-Methoxy-4-azatricyclo[5.2.2.0^{2.6}]undec-8-en-3,5-dion in 100 ml absolutem Tetrahydrofuran und rührt über Nacht unter Rückfluß. Man zersetzt mit je 6 ml Wasser, 15 %iger KOH-Lösung und wieder Wasser, saugt die anorganischen Salze ab, kocht sie zweimal mit Tetrahydrofuran aus, engt die Filtrate ein und destilliert den Rückstand.
Ausbeute: 12,3 g (68,6 % der Theorie),
Siedepunkt: 100°C/0,04 mbar.

### Beispiel Z 2

### A. 1-Ethoxycarbonyl-4-azatricyclo[5.2.2.0^{2.6}]undec-8-en-3,5-dion

Man erhitzt 13,5 g (0,138 mol) Maleinimid und 25,3 g (0,17 mol) 1,3-Cyclohexadien-1-carbonsäureethylester mit 1,4 g 4-(tert.-Butyl)-brenzkatechin in 275 ml Toluol über Nacht unter Rückfluß. Man engt ein und kristallisiert aus Isopropanol um.
Ausbeute: 25,9 g (75 % der Theorie),
Schmelzpunkt: 172-173°C.

### B. 1-Hydroxymethyl-4-azatricyclo[5.2.2.0^{2.6}]undec-8-en

12,5 g (50 mmol) 1-Ethoxycarbonyl-4-azatricyclo-[5.2.2.0^{2.6}]undec-8-en-3,5-dion werden wie unter Z 1-B beschrieben mit LiAlH₄ reduziert und entsprechend aufgearbeitet.
Ausbeute: 2,3 g (25,6 % der Theorie),
Siedepunkt: 122-124°C/0,5 mbar.

### Beispiel Z 3

### A. 1-Dimethylamino-4-azatricyclo[5.2.2.0^{2.6}]undec-8-en-3,5-dion

Man rührt 9 g (73 mmol) 1-Dimethylaminocyclohexa-1,3-dien und 6,4 g (66 mmol) Maleinimid in 100 ml absolutem Dioxan über Nacht bei Raumtemperatur. Man engt ein und kristallisiert den Rückstand aus Isopropanol um.
Ausbeute: 12 g (82,5 % der Theorie),
Schmelzpunkt: 170-172°C.

### B. 1-Dimethylamino-4-azatricyclo[5.2.2.0.^{2.6}]undec-8-en

12 g (54,5 mmol) 1-Dimethylamino-4-azatricyclo [5.2.2.0^{2.6}]undec-8-en-3,5-dion werden wie unter Z 1-B beschrieben mit LiAlH₄ reduziert und entsprechend aufgearbeitet.
Ausbeute: 7,5 g (71,5 % der Theorie),
Siedepunkt: 91-93°C/0,04 mbar.

### Beispiel Z 4

### A. 1-Cyano-4-azatricyclo[5.2.2.0^{2.6}]undec-8-en-3,5-dion

Man erhitzt 59 g (0,56 mol) 1-Cyano-cyclohexa-1,3-dien mit 54 g (0,56 mol) Maleinimid und 5 g 4-(tert.-Butyl)-brenzkatechin in 1000 ml Dimethylformamid 8 Stunden auf 150°C. Man engt ein, verrührt mit Wasser und Toluol, saugt das kristallisierte Produkt ab, wäscht es mit Isopropanol und trocknet an der Luft.
Ausbeute: 71 g (67,5 % der Theorie),
Schmelzpunkt: 213°C.

### B. 1-Aminomethyl-4-azatricyclo[5.2.2.0^{2.6}]undec-8-en

15,4 g (76,2 mmol) 1-Cyano-4-azatricyclo[5.2.2.0^{2.6}]undec-8-en-3,5-dion werden wie unter Z 1-B beschrieben mit LiAlH₄ reduziert und entsprechend aufgearbeitet.
Ausbeute: 7 g (51,5 % der Theorie),
Siedepunkt: 97°C/0,1 mbar.

### Beispiel Z 5

### A. 4-Azatricyclo[5.2.2.0^{2.6}]undec-8-en-3,5-dion-1-carbonsäureamid

Man löst 41 g (0,203 mol) 1-Cyano-4-azatricyclo[5.2.2.0^{2.6}]undec-8-en-3,5-dion in 150 ml 10 %iger Natronlauge und tropft bei 0°C 130 ml 30 %iges H₂O₂ hinzu. Man rührt noch 30 Minuten bei 0°C, dann 3 Stunden bei Raumtemperatur, stellt mit Essigsäure auf pH 4,5 ein und saugt das auskristallisierende Produkt ab.
Ausbeute: 36,3 g (81 % der Theorie),
Schmelzpunkt: 158°C (aus Isopropanol).

### B. 1-Amino-4-azatricyclo[5.2.2.0^{2.6}]undec-8-en-3,5-dion

Man erhitzt 74,5g (0,338 mol) 4-Azatricyclo[5.2.2.0^{2.6}]undec-8-en-3,5-dion-1-carbonsäureamid mit 139 g (0,354 mol) J-Hydroxy-J-tosyloxy-jodbenzol in 1000 ml Acetonitril 3 Stunden unter Rückfluß. Man kühlt ab und saugt das auskristallisierte Toluolsulfonsäuresalz des Produktes ab.
Ausbeute: 112 g (95 % der Theorie),
Schmelzpunkt: 255-256°C.

Das Salz wird in 300 ml Wasser suspendiert und eine Lösung von 12,5 g NaOH in 40 ml Wasser zugesetzt. Das Salz geht rasch in Lösung und nach einiger Zeit kristallisiert das Produkt aus. Ausbeute: 43,5 g (70 % der Theorie).

### C. 1-Amino-4-azatricyclo[5.2.2.0^{2.6}]undec-8-en

45 g (0,234 mol) 1-Amino-4-azatricyclo[5.2.2.0^{2.6}]undec-8-en-3,5-dion werden mit 24 g LiAlH₄ in 500 ml absolutem Dioxan wie unter Z 1-B reduziert und entsprechend aufgearbeitet.
Ausbeute: 14,8 g (40 % der Theorie)
Siedepunkt: 95-105°C/0,08 mbar

### Beispiel Z 6

### A. 4-Benzyl-1-cyano-4-azatricyclo[5.2.2.0^{2.6}]undec-8-en-3,5-dion

Man erhitzt 39 g (0,35 mol) 1-Cyano-cyclohexa-1,3-dien mit 65,5 g (0,35 mol) N-Benzyl-maleinimid in 700 ml Xylol eine Stunde auf 100°C, dann 1 Stunde unter Rückfluß. Nach dem Abkühlen saugt man die Kristalle ab und kristallisiert aus Xylol um.
Ausbeute: 75 g (73,3 % der Theorie),
Schmelzpunkt: 175-177°C.

### B. 1-Aminomethyl-4-benzyl-4-azatricyclo[5.2.2.0^{2.6}]undec-8-en

14,6 g (50 mmol) 4-Benzyl-1-cyano-4-azatricyclo-[5.2.2.0^{2.6}]undec-8-en-3,5-dion werden mit 5 g LiAlH₄ wie unter Z 1-B beschrieben reduziert und entsprechend aufgearbeitet.
Ausbeute: 6,4 g (47,7 % der Theorie),
Siedepunkt: 158°C/0,1 mbar.

### C. 1-Aminomethyl-4-azatricyclo[5.2.2.0^{2.6}]undecan

7,4g (27,5 mmol) 1-Aminomethyl-4-benzyl-4-azatricyclo[5.2.2.0^{2.6}]undec-8-en werden in 100 ml Tetrahydrofuran mit 1,5 g Palladium-Aktivkohle bei 100°C/100 bar hydriert. Der Katalysator wird abgesaugt und der Rückstand destilliert.
Ausbeute: 3,1 g (62,5 % der Theorie),
Siedepunkt: 89°C/0,1 mbar.

### Beispiel Z 7

### A. 4-Benzyl-4-azatricyclo[5.2.2.0^{2.6}]undec-8-en-3,5-dion-1-carbonsäureamid

Man löst 14,5 g (50 mmol) 4-Benzyl-1-cyano-4-azatricyclo[5.2.2.0^{2.6}]undec-8-en-3,5-dion in 25 ml Methylenchlorid, setzt 2,5 g Tetrabutylammoniumhydrogensulfat und 17,5 ml 20 %ige Natronlauge hinzu und tropft bei 0°C 24 ml 30 %iges H₂O₂ hinzu. Man rührt 30 Minuten bei 0°C, dann über Nacht bei Raumtemperatur, verdünnt mit Methylenchlorid, trennt die organische Phase ab und wäscht sie mit Kochsalzlösung. Man trocknet über MgSO₄, engt ein und kocht das kristalline Produkt mit Xylol aus, wobei eventuell nicht umgesetztes Edukt in Lösung geht. Das Produkt wird heiß abgesaugt und an der Luft getrocknet.
Ausbeute: 13 g (83,8 % der Theorie),
Schmelzpunkt: 224°C.

### B. 1-Amino-4-benzyl-4-azatricyclo[5.2.2.0^{2.6}]undec-8-en-3,5-dion

17,6 g (56,7 mmol) 4-Benzyl-4-azatricyclo[5.2.2.0^{2.6}]undec-8-en-3,5-dion-1-carbonsäureamid werden mit 22,2 g J-Hydroxy-J-tosyloxy-jodbenzol wie unter Z 5-B beschrieben zur Reaktion gebracht und entsprechend aufgearbeitet.
Ausbeute: 22,5 g (87 % der Theorie) des Tosylates,
Schmelzpunkt: 222°C.

Hieraus wird wie unter Z 5-B beschrieben die freie Base hergestellt.
Ausbeute: 12 g (86 % der Theorie),
Schmelzpunkt: 130-134°C

### C. 1-Amino-4-benzyl-4-azatricyclo[5.2.2.0^{2.6}]undec-8-en

19,3 g (68,2 mmol) 1-Amino-4-benzyl-4-azatricyclo[5.2.2.0^{2.6}]undec-8-en-3,5-dion werden mit 8 g LiAlH₄ wie unter Z 1-B beschrieben reduziert und entsprechend aufgearbeitet.
Ausbeute: 12,6 g (72,6 % der Theorie),
Siedepunkt: 145-148°C/0,15 mbar,
Schmelzpunkt: 59-61°C.

### D. 1-Amino-4-azatricyclo[5.2.2.0^{2.6}]undecan

Man hydriert 8,3 g (32,6 mmol) 1-Amino-4-benzyl-4-azatricyclo[5.2.2.0^{2.6}]undec-8-en in 100 ml Ethanol an 1 g Palladium-Aktivkohle bei 100°C/100 bar. Man saugt den Katalysator ab, engt das Filtrat ein und destilliert den Rückstand.
Ausbeute: 4,4 g ( 81,2 % der Theorie),
Siedepunkt: 115°C/0,05 mbar.

### Beispiel Z 8

### A. 4-Benzyl-3,5-dioxo-4-azatricyclo[5.2.1.0^{2.6}]dec-8-en-1-carbonsäureethylester

Man erhitzt 32,8 g (0,17 mol) N-Benzylmaleinimid mit 24,2 g (0,17 mol) Tricyclo[5.2.1.0^{2.6}]deca-3,8-dien-1,4-dicarbonsäurediethylester in 200 ml 1,4-Dioxan über Nacht unter Rückfluß. Man engt ein, nimmt in 80 ml Isopropanol auf und laßt auskristallisieren.
Ausbeute: 23 g (41,6 % der Theorie),
Schmelzpunkt: 78-80°C.

### B. 4-Benzyl-3,5-dioxo-4-azatricyclo[5.2.1.0^{2.6}]dec-8-en-1-carbonsäure

Man erhitzt 30 g (90 mmol) 4-Benzyl-3,5-dioxo-4-azatricyclo[5.2.1.0^{2.6}]dec-8-en-1-carbonsäureethylester mit 5,3 g (0,13 mol) NaOH in 150 ml Methanol über Nacht unter Rückfluß. Man engt ein, nimmt in 150 ml Wasser auf und stellt mit Salzsäure sauer. Das auskristallisierende Produkt wird abgesaugt, mit Wasser gewaschen und an der Luft getrocknet.
Ausbeute: 23,5 g (87,8 % der Theorie),
Schmelzpunkt: 157°C.

### C. 4-Benzyl-3,5-dioxo-4-azatricyclo[5.2.1.0^{2.6}]dec-8-en-1-carbonsäureamid

Zu 3 g (10 mmol) 4-Benzyl-3,5-dioxo-4-azatricyclo[5.2.1.0^{2.6}]dec-8-en-1-carbonsäure in 20 ml absolutem Tetrahydrofuran tropft man bei 0°C 1,1 g (11 mmol) Triethylamin und anschließend 1,15 g (12 mmol) Chlorameisensäuremethylester. Man rührt noch eine Stunde bei 0°C und gießt die Lösung auf 20 ml Ammoniaklösung. Man rührt 1 Stunde bei Raumtemperatur, extrahiert mehrfach mit Chloroform, trocknet die Extrakte über MgSO₄ und engt ein, worauf das Produkt kristallisiert.
Ausbeute: 1,78 g (60 % der Theorie),
Schmelzpunkt: 168°C.

### Herstellung der Wirkstoffe

### Beispiel 1

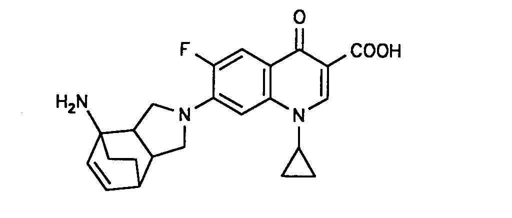

265 mg (1 mmol) 1-Cyclopropyl-6,7-difluor-1,4-dihydro-4-oxo-3-chinolincarbonsäure werden in einer Mischung aus 4 ml Acetonitril und 2 ml Dimethylformamid mit 170 mg (1,5 mmol) 1,4-Diazabicyclo[2.2.2]octan und 210 mg (1,1 mmol) 86 %-iges 4-Aza-tricyclo[5.2.2.0^{2,6}]undec-8-en-1-ylamin 1 Stunde unter Rückfluß erhitzt. Die Mischung wird eingeengt, der Rückstand mit 40 ml Wasser verrührt (pH = 7), der ausgefallene Niederschlag wird abgesaugt, mit Wasser gewaschen und bei 80°C im Hochvakuum getrocknet.
Ausbeute: 286 mg (70 % der Theorie) 7-(1-Amino-4-aza-tricyclo[5.2.2.0^{2,6}]undec-8-en-4-yl)-1-cyclopropyl-6-fluor-1,4- dihydro-4-oxo-3-chinolincarbonsäure,
Schmelzpunkt: 272-274°C (unter Zersetzung).

### Beispiel 2

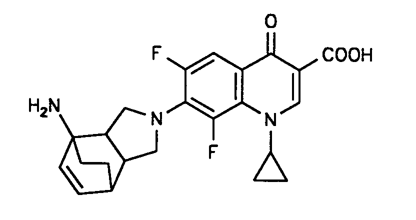

Unter entsprechenden Bedingungen wie in Beispiel 1 erhält man mit 1-Cyclopropyl-6,7,8-trifluor-1,4-dihydro-4-oxo-3-chinolincarbonsäure in 61 %-iger Ausbeute 7-(1-Amino-4-aza-tricyclo[5.2.2.0^{2,6}]undec-8-en-4-yl)-1-cyclopropyl-6,8-difluor-1,4-dihydro-4-oxo-3-chinolincarbonsäure,
Schmelzpunkt: 226-227°C (unter Zersetzung).

### Beispiel 3

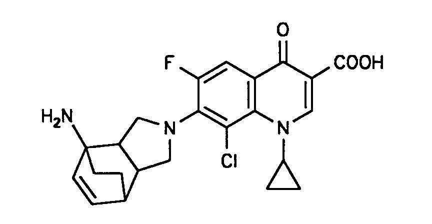

Unter entsprechenden Bedingungen wie in Beispiel 1 erhält man mit 8-Chlor-1-cyclopropyl-6,7-difluor-1,4-dihydro-4-oxo-3-chinolincarbonsäure ein Rohprodukt, das an Kieselgel chromatographisch gereinigt wird (Laufmittel: Dichlormethan/17 %-iges wäßriges Ammoniak/Methanol 30:8:1). Man erhält in 72 %-iger Ausbeute 7-(1-Amino-4-aza-tricyclo[5.2.2.0^{2,6}]undec-8-en-4-yl)-8-chlor-1- cyclopropyl-6-fluor-1,4-dihydro-4-oxo-3-chinolincarbonsäure,
Schmelzpunkt: 218-229°C (unter Zersetzung).

### Beispiel 4

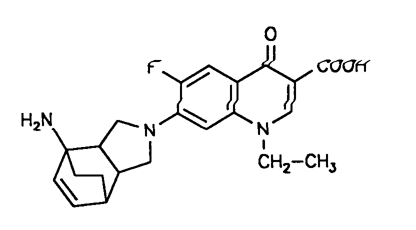

Unter entsprechenden Bedingungen wie in Beispiel 1 erhält man mit 1-Ethyl-6,7-difluor-1,4-dihydro-4-oxo-3-chinolincarbonsäure ein Rohprodukt, das an Kieselgel chromatographisch gereinigt wird (Laufmittel: Dichlormethan/17 %-iges wäßriges Ammoniak/Methanol 30:8:1). Man isoliert in 53 %-iger Ausbeute 7-(1-Amino-4-azatricyclo[5.2.2.0^{2,6}]undec-8-en-4-yl)-1-ethyl-6-fluor-1,4- dihydro-4-oxo-3-chinolincarbonsäure, deren Schmelzpunkt (Sintern ab 75°C) nicht ermittelt werden kann. ¹H-NMR (CF₃COOD): δ 6,41 d (1 H) und 6,63 ppm "t" (1 H).

### Beispiel 5

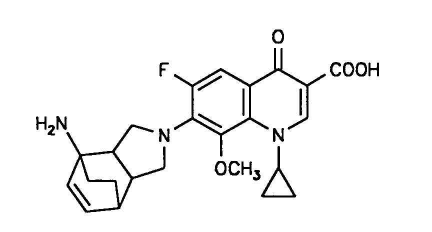

Unter entsprechenden Bedingungen wie in Beispiel 1 erhält man mit 1-Cyclopropyl-6,7-difluor-1,4-dihydro-8-methoxy-4-oxo-3- chinolincarbonsäurein64%-iger Ausbeute 7-(1-Amino-4-aza-tricyclo[5.2.2.0^{2,6}]undec-8-en-4-yl)-1-cyclopropyl-6-fluor-1,4-dihydro-8-methoxy-4-oxo-3-chinolincarbonsäure,
Schmelzpunkt: 224-225°C (unter Zersetzung).

### Beispiel 6

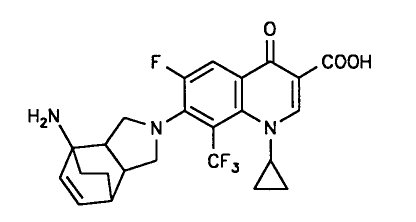

Unter entsprechenden Bedingungen wie in Beispiel 1 erhält man mit 1-Cyclopropyl-6,7-difluor-1,4-dihydro-4-oxo-8-trifluormethyl-3- chinolincarbonsäure ein Rohprodukt, das an Kieselgel chromatographisch gereinigt wird (Laufmittel: Dichlormethan/17 %-iges wäßriges Ammoniak/Methanol 30:8:1). Man isoliert in 32 %-iger Ausbeute 7-(1-Amino-4-aza-tricyclo[5.2.2.0^{2,6}]undec-8-en-4-yl)-1-cyclopropyl-6- fluor-1,4-dihydro-4-oxo-8-trifluormethyl-3-chinolincarbonsäure,
Schmelzpunkt: 163-166°C (unter Zersetzung).

### Beispiel 7

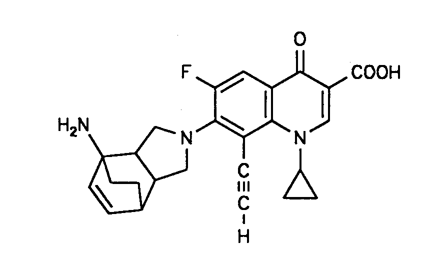

Unter entsprechenden Bedingungen wie in Beispiel 1 erhält man mit 1-Cyclopropyl-8-ethinyl-6,7-difluor-1,4-dihydro-4-oxo-3- chinolincarbonsäure in 87 %-iger Ausbeute 7-(1-Amino-4-aza-tricyclo[5.2.2.0^{2,6}]undec-8-en-4-yl)-1-cyclopropyl-8-ethinyl-6-fluor-1,4-dihydro-4-oxo-3-chinolincarbonsäure, deren Schmelzpunkt wegen vorzeitigen Sinterns ab 107°C nicht ermittelt werden kann.
¹H-NMR (CDCl₃): δ 3,84 s (1 H), 6,03 d (1 H), 6,20 ppm dd (1 H).

### Beispiel 8

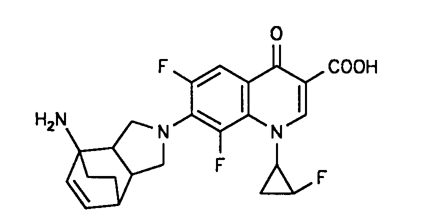

Unter entsprechenden Bedingungen wie in Beispiel 1 erhält man mit 6,7,8-Trifluor-1-(cis-2-fluorcyclopropyl)-1,4-dihydro-4-oxo-3- chinolincarbonsäureeinRohprodukt,das an Kieselgel chromatographisch gereinigt wird (Laufmittel: Dichlormethan/17 %-iges wäßriges Ammoniak/Methanol 30:8:1). Man isoliert in 55 %-iger Ausbeute 7-(1-Amino-4-aza-tricyclo[5.2.2.0^{2,6}]undec-8-en-4-yl)-6,8-difluor-1-(cis- 2-fluorcyclopropyl)-1,4-dihydro-4-oxo-3-chinolincarbonsäure,
Schmelzpunkt: 222-225°C (unter Zersetzung).

### Beispiel 9

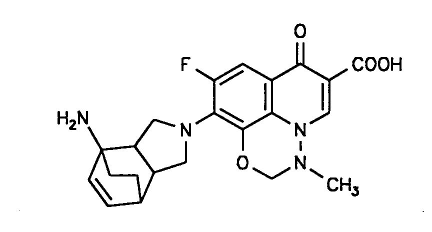

150 mg (0.53 mmol) 9,10-Difluor-3-methyl-7-oxo-2,3-dihydro-7H-pyrido[1,2,3-d,e][1,3,4]-benzoxadiazin-6-carbonsäure werden mit 130 mg (0.79 mmol) 4-Azatricyclo[5.2.2.0^{2,6}]undec-8-en-1-ylamin in 5 ml Pyridin 8 Stunden unter Argon auf 110°C erwärmt. Die Mischung wird im Hochvakuum eingeengt, der Rückstand aus Ethanol umkristallisiert und getrocknet.
Ausbeute: 55 mg(24 % der Theorie) 10-(1-Amino-4-azatricyclo[5.2.2.0^{2,6}]undec-8-en-4-yl)-9-fluor-3-methyl-7-oxo- 2,3-dihydro-7H-pyrido[1,2,3-d,e][1,3,4]benzoxadiazin-6-carbonsäure,
Schmelzpunkt: 215-223°C (unter Zersetzung).

### Beispiel 10

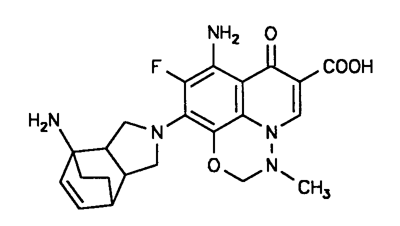

150 mg (0.51 mmol) 8-Amino-9,10-difluor-3-methyl-7-oxo-2,3-dihydro-7H-pyrido[1,2,3-d,e][1,3,4]benzoxadiazin-6-carbonsäure werden mit 125 mg (0.76 mmol) 4-Azatricyclo[5.2.2.0^{2,6}]undec-8-en-1-ylamin in 4.5 ml Dimethylsulfoxid drei Stunden unter Argon auf 120°C erwärmt. Die Mischung wird im Hochvakuum eingeengt, der Rückstand aus Ethanol umkristallisiert und getrocknet.
Ausbeute: 190 mg (85 % der Theorie) 8-Amino-10-(1-amino-4-azatricyclo[5.2.2.0^{2,6}]-undec-8-en-4-yl)-9-fluor-3-methyl-7-oxo-2,3- dihydro-7H-pyrido[1,2,3-d,e][1,3,4]-benzoxadiazin-6-carbonsäure,
Schmelzpunkt: 230-239°C (unter Zersetzung).

### Beispiel 11

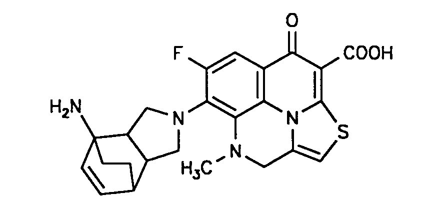

350 mg (1.086 mmol) 7,8-Difluor-5-oxo-9,1-[(N-methylimino)methano]-5H-thiazolo-[3,2-a]-chinolin-4-carbonsäure werden mit 267 mg (1.63 mmol) 4-Azatricyclo[5.2.2.0^{2,6}]undec-8-en-1-ylamin in 8 ml Dimethylsulfoxid sechs Stunden unter Argon auf 120°C erwärmt. Die Mischung wird im Hochvakuum eingeengt, der Rückstand aus Ethanol umkristallisiert und getrocknet.
Ausbeute: 330 mg (65 % der Theorie) 8-(1-Amino-4-azatricyclo[5.2.2.0^{2,6}]undec-8-en-4-yl)-7-fluor-5-oxo-9,1-[(N- methyl-imino)methano]-5H-thiazolo[3,2-a]chinolin-4-carbonsäure,
Schmelzpunkt: > 300°C.

### Beispiel 12

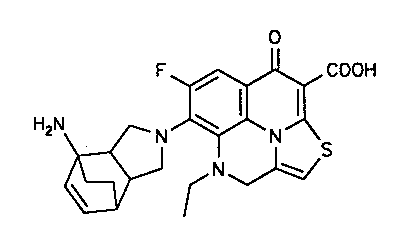

Analog zum Beispiel 11 wird bei der Umsetzung mit 7,8-Difluor-5-oxo-9,1-[(N-ethylimino)methano]-5H-thiazolo[3,2-a]chinolin-4-carbonsäure 8-(1-Amino-4-azatricyclo[5.2.2.0^{2,6}]undec-8-en-4-yl)-7-fluor-5-oxo-9,1-[(N-ethyl-imino)methano]-5H-thiazolo[3,2-a]chinolin-4-carbonsäure erhalten.
Schmelzpunkt: > 300°C.

### Beispiel 13

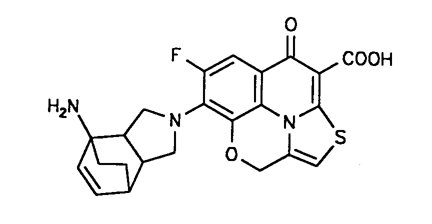

100 mg (0.323 mmol) 7,8-Difluor-5-oxo-9,1-(epoxymethano)-5H-thiazolo[3,2-a]-chinolin-4-carbonsäure werden mit 80 mg (0.49 mmol) 4-Azatricyclo[5.2.2.0^{2,6}]undec-8-en-1-ylamin in 3 ml DMSO 2 Stunden unter Argon auf 100°C erwärmt. Die Mischung wird im Hochvakuum eingeengt, der Rückstand aus Ethanol umkristallisiert und getrocknet.
Ausbeute: 114 mg (78 % der Theorie) 8-(1-Amino-4-azatricyclo[5.2.2.0^{2,6}]undec-8-en-4-yl)-7-fluor-5-oxo-9,1- (epoxymethano)-5H-thiazolo[3,2-a]chinolin-4-carbonsäure,
Schmelzpunkt: 254-263°C.

### Beispiel 14

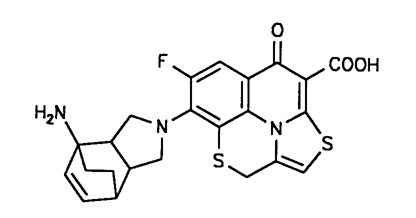

100 mg (0.307 mmol) 7,8-Difluor-5-oxo-9,1-(epithiomethano)-5H-thiazolo[3,2-a]-chinolin-4-carbonsäure werden mit 76 mg (0.46 mmol) 1-Amino-4-azatricyclo[5.2.2.0^{2,6}]undec-8-en in 3 ml DMSO vier Stunden unter Argon auf 120°C erwärmt. Die Mischung wird im Hochvakuum eingeengt, der Rückstand aus Ethanol umkristallisiert und getrocknet.
Ausbeute: 119 mg (83 % der Theorie) 8-(1-Amino-4-azatricyclo[5.2.2.0^{2,6}]undec-8-en-4-yl)-7-fluor-5-oxo-9,1- (epithiomethano)-5H-thiazolo[3,2-a]chinolin-4-carbonsäure,
Schmelzpunkt: 285-290°C.

### Beispiel 15

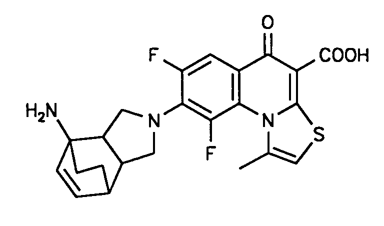

80 mg (0.254 mmol) 7,8-Difluor-1-methyl-5-oxo-5H-thiazolo[3,2-a]-chinolin-4-carbonsäure werden mit 46 mg (0.28 mmol) 4-Azatricyclo[5.2.2.0^{2,6}]undec-8-en-1-ylamin in 3 ml DMSO 3 Stunden unter Argon auf 80°C erwärmt. Die Mischung wird im Hochvakuum eingeengt, der Rückstand aus Ethanol umkristallisiert und getrocknet.
Ausbeute: 41 mg (35 % der Theorie) 8-(1-Amino-4-azatricyclo[5.2.2.0^{2,6}]undec-8-en-4-yl)-7-fluor-1-methyl-5-oxo-5H-thiazolo[3,2-a]chinolin-4-carbonsäure,
Schmelzpunkt: 122°C.

### Beispiel 16

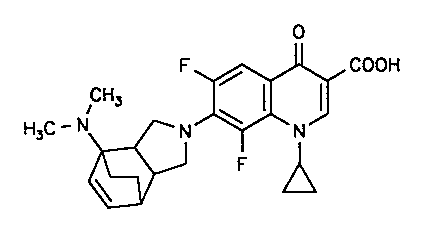

283 mg (1 mmol) 1-Cyclopropyl-6,7,8-trifluor-1,4-dihydro-4-oxo-3-chinolincarbonsäure werden in einer Mischung aus 2 ml Acetonitril und 1 ml Dimethylformamid mit 224 mg (2 mmol) 1,4-Diazabicyclo[2.2.2]octan und 210 mg (1,1 mmol) (4-Azatricyclo[5.2.2.0^{2,6}]undec-8-en-1-yl)-dimethyl-amin 1 Stunde unter Rückfluß erhitzt. Die Mischung wird eingeengt, der Rückstand mit Wasser verrührt, der ausgefallene Niederschlag wird abgesaugt, mit Wasser gewaschen und bei 100°C im Hochvakuum getrocknet.
Ausbeute: 360 mg (79 % der Theorie) 1-Cyclopropyl-7-(1-dimethylamino-4-azatricyclo[5.2.2.0^{2,6}]undec-8-en-4-yl)-6,8-difluor-1,4-dihydro-4-oxo-3- chinolincarbonsäure,
Schmelzpunkt: 238-240°C (unter Zersetzung).

### Beispiel 17

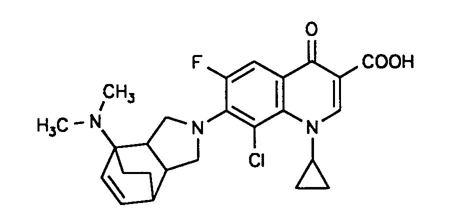

Unter entsprechenden Bedingungen wie in Beispiel 16 erhält man mit 8-Chlor-1-cyclopropyl-6,7-difluor-1,4-dihydro-4-oxo-3-chinolincarbonsäure in 65 %-iger Ausbeute 8-Chlor-1-cyclopropyl-7-(1-dimethylamino-4-azatricyclo[5.2.2.0^{2,6}]undec-8-en-4-yl)-6-fluor-1,4-dihydro-4-oxo-3- chinolincarbonsäure,
Schmelzpunkt: 180-183°C (unter Zersetzung).

### Beispiel 18

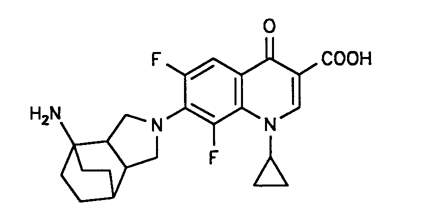

283 mg (1 mmol) 1-Cyclopropyl-6,7,8-trifluor-1,4-dihydro-4-oxo-3-chinolincarbonsäure werden in einer Mischung aus 4 ml Acetonitril und 2 ml Dimethylformamid mit 224 mg (2 mmol) 1,4-Diazabicyclo[2.2.2]octan und 191 mg (1,1 mmol) 4-Azatricyclo[5.2.2.0^{2,6}]undec-1-ylamin 1 Stunde unter Rückfluß erhitzt. Die Mischung wird bei 70°C/15 mbar eingeengt, der Rückstand mit Wasser verrührt, der ausgefallene Niederschlag wird abgesaugt, mit Wasser gewaschen und bei 100°C im Hochvakuum getrocknet.
Ausbeute: 303 mg (73 % der Theorie) 7-(1-Amino-4-azatricyclo[5.2.2.0^{2,6}]undec-4-yl)-1-cyclopropyl-6,8-difluor- 1,4-dihydro-4-oxo-3-chinolincarbonsäure,
Schmelzpunkt: 240-245°C (unter Zersetzung).

### Beispiel 19

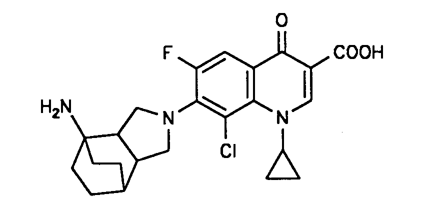

Unter entsprechenden Bedingungen wie in Beispiel 18 erhält man mit 8-Chlor-1-cyclopropyl-6,7-difluor-1,4-dihydro-4-oxo-3-chinolincarbonsäure in 93 %-iger Ausbeute 7-(1-Amino-4-azatricyclo[5.2.2.0^{2,6}]undec-4-yl)-8-chlor-1-cyclopropyl-6-fluor-1,4-dihydro-4-oxo-3-chinolincarbonsäure,
Schmelzpunkt: 232-235°C (unter Zersetzung).

### Beispiel 20

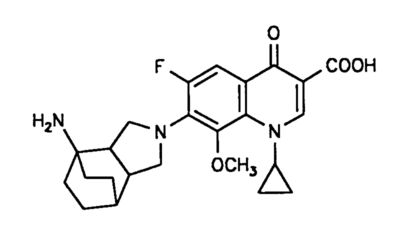

295 mg(1 mmol)1-Cyclopropyl-6,7-difluor-1,4-dihydro-8-methoxy-4-oxo-3-chinolincarbonsäure werden in 3 ml Acetonitril mit 208 mg (2 mmol) Trimethylborat, 123 mg (1,1 mmol) 1,4-Diazabicyclo[2.2.2]octan und 185 mg (1,1 mmol) 4-Aza-tricyclo[5.2.2.0^{2,6}]undec-1-ylamin bei Raumtemperatur versetzt und die Mischung anschließend 4 Stunden unter Rückfluß erhitzt. Das Reaktionsgemisch wird bei 80°C/20 mbar eingeengt, der Rückstand mit Wasser verrührt, der ausgefallene Niederschlag wird abgesaugt, mit Wasser gewaschen und bei 100°C im Hochvakuum getrocknet.
Ausbeute: 218 mg (51 % der Theorie) 7-(1-Amino-4-azatricyclo[5.2.2.0^{2,6}]undec-4-yl)-1-cyclopropyl-6-fluor-1,4- dihydro-8-methoxy-4-oxo-3-chinolincarbonsäure,
Schmelzpunkt: 234-237°C (unter Zersetzung).

### Beispiel 21

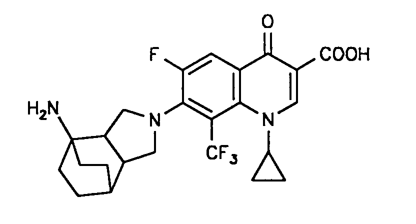

Unter entsprechenden Bedingungen wie in Beispiel 18 erhält man mit 1-Cyclopropyl-6,7-difluor-1,4-dihydro-4-oxo-8-trifluormethyl-3- chinolincarbonsäureeinRohprodukt, aus dem nach chromatographischer Reinigung an Kieselgel ((Laufmittel: Dichlormethan/17 %-iges wäßriges Ammoniak/Methanol 30:8:1) in 34 %-iger Ausbeute 7-(1-Amino-4-azatricyclo[5.2.2.0^{2,6}]undec-4-yl)-1-cyclopropyl-6-fluor-1,4- dihydro-4-oxo-8-trifluormethyl-3-chinolincarbonsäure isoliert wurde.
Schmelzpunkt: 211-215°C (unter Zersetzung).

### Beispiel 22

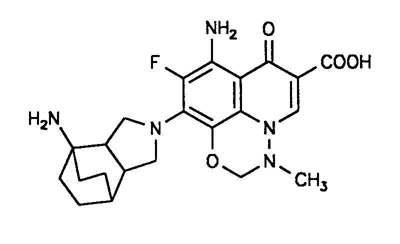

100 mg (0.336 mmol) 8-Amino-9,10-difluor-3-methyl-7-oxo-2,3-dihydro-7H-pyrido[1,2,3-d,e][1,3,4]benzoxadiazin-6-carbonsäure werden mit 111 mg (0.67 mmol) 4-Azatricyclo[5.2.2.0^{2,6}]undecan-1-ylamin in 3 ml Dimethylsulfoxid 4 Stunden unter Argon auf 120°C erwärmt. Die Mischung wird im Hochvakuum eingeengt, der Rückstand aus Ethanol umkristallisiert und getrocknet.
Ausbeute: 105 mg (70 % der Theorie) 8-Amino-10-(1-amino-4-azatricyclo[5.2.2.0^{2,6}]undecan-4-yl)-9-fluor-3-methyl-7-oxo-2,3-dihydro- 7H-pyrido[1,2,3-d,e][1,3,4]benzoxadiazin-6-carbonsäure,
Schmelzpunkt: 236°C.

### Beispiel 23

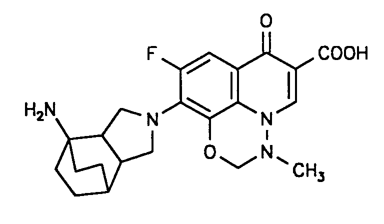

Analog zum Beispiel 22 wird bei der Umsetzung mit 9,10-Difluor-3-methyl-7-oxo-2,3-dihydro-7H-pyrido[1,2,3-d,e][1,3,4]benzoxadiazin-6- carbonsäure-10-(1-Amino-4-azatricyclo[5.2.2.0^{2,6}]undecan-4-yl)-9-fluor-3-methyl-7-oxo-2,3-dihydro-7H-pyrido[1,2,3-d,e][1,3,4]benzoxadiazin-6- carbonsäure erhalten.
Schmelzpunkt: 143°C.

### Beispiel 24

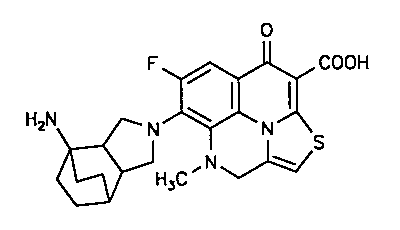

150 mg (0.465 mmol) 7,8-Difluor-5-oxo-9,1-[(N-methylimino)methano]-5H-thiazolo[3,2-a]-chinolin-4-carbonsäure werden mit 153 mg (0.92 mmol) 4-Aza-tricyclo[5.2.2.0^{2,6}]undecan-1-yl in 3 ml Dimethylsulfoxid 3 Stunden unter Argon auf 130°C erwärmt. Die Mischung wird im Hochvakuum eingeengt, der Rückstand aus Ethanol umkristallisiert und getrocknet.
Ausbeute: 137 mg (63 % der Theorie) 8-(1-Amino-4-azatricyclo[5.2.2.0^{2,6}]undecan-4-yl)-7-fluor-5-oxo-9,1-[(N- methyl-imino)methano]-5H-thiazolo[3,2-a]chinolin-4-carbonsäure,
Schmelzpunkt: >300°C.

### Beispiel 25

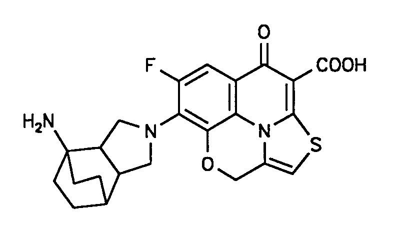

Analog zum Beispiel 13 wird bei der Umsetzung mit 1-Amino-4-azatricyclo[5.2.2.0^{2,6}]undecan 8-(1-Amino-4-azatricyclo[5.2.2.0^{2,6}]undecan-4-yl)-7-fluor-5-oxo-9,1-(epoxymethano)-5H-thiazolo[3,2-a]chinolin-4-carbonsäure erhalten.
Schmelzpunkt: 270°C.

### Beispiel 26

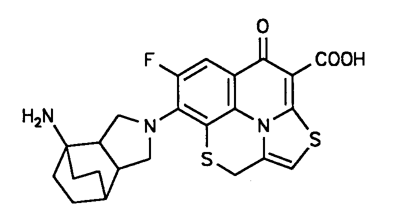

Analog zum Beispiel 14 wird bei der Umsetzung mit 1-Amino-4-azatri-cyclo[5.2.2.0^{2,6}]undecan 8-(1-Amino-4-azatricyclo[5.2.2.0^{2,6}]undecan-4-yl)-7-fluor-5-oxo-9,1-(epithiomethano)-5H-thiazolo[3,2-a]chinolin-4-carbonsäure erhalten.
Schmelzpunkt: 270°C (unter Zersetzung).

### Beispiel 27

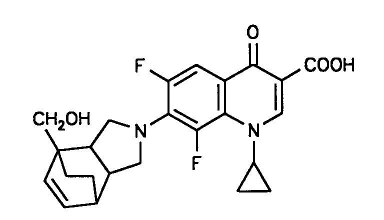

283 mg (1 mmol) 1-Cyclopropyl-6,7,8-trifluor-1,4-dihydro-4-oxo-3-chinolincarbonsäure werden in einer Mischung aus 4 ml Acetonitril und 2 ml Dimethylformamid mit 224 mg (2 mmol) 1,4-Diazabicyclo[2.2.2]octan und 200 mg (1,1 mmol) (4-Azatricyclo[5.2.2.0^{2,6}]undec-8-en-1-yl)-methanol 1 Stunde unter Rückfluß erhitzt. Der ausgefallene Niederschlag wird abgesaugt, mit Wasser gewaschen und bei 80°C im Hochvakuum getrocknet.
Ausbeute: 360 mg (81 % der Theorie) 1-Cyclopropyl-6,8-difluor-1,4-dihydro-7-(1-hydroxymethyl-4-azatricyclo[5.2.2.0^{2,6}]undec-8-en-4-yl)-4- oxo-3-chinolincarbonsäure,
Schmelzpunkt: 242-244°C (unter Zersetzung).

### Beispiel 28

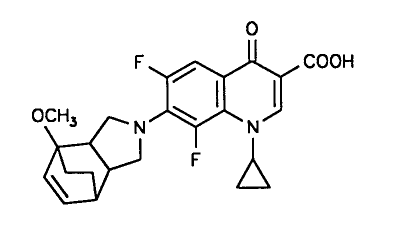

566 mg (2 mmol) 1-Cyclopropyl-6,7,8-trifluor-1,4-dihydro-4-oxo-3-chinolincarbonsäure werden in einer Mischung aus 8 ml Acetonitril und 4 ml Dimethylformamid mit 350 mg (3,1 mmol) 1,4-Diazabicyclo[2.2.2]octan und 380 mg (2,1 mmol) 1-Methoxy-4-aza-tricyclo[5.2.2.0^{2,6}]undec-8-en 1 Stunde unter Rückfluß erhitzt. Die Mischung wird eingeengt, der Rückstand mit 10 ml Wasser verrührt (pH = 7), der ausgefallene Niederschlag wird abgesaugt, mit Wasser gewaschen und bei 90°C im Hochvakuum getrocknet. Man erhält 821 mg eines Rohproduktes, das aus Glykolmonomethylether umkristallisiert, mit Ethanol gewaschen und bei 120°C im Hochvakuum getrocknet wird.
Ausbeute: 676 mg (76 % der Theorie) 1-Cyclopropyl-6,8-difluor-1,4-dihydro-7-(1-methoxy-4-azatricyclo[5.2.2.0^{2,6}]undec-8-en-4-yl)-4-oxo-3- chinolincarbonsäure,
Schmelzpunkt: 183-185°C (unter Zersetzung).

### Beispiel 29

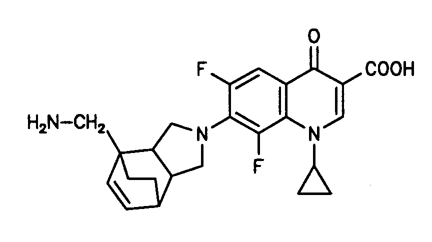

Unter entsprechenden Bedingungen wie in Beispiel 18 erhält man mit (4-Azatricyclo[5.2.2.0^{2,6}]undec-8-en-1-ylmethyl)amin nach Umkristallisation aus Glykolmonomethylether in 59 %-iger Ausbeute 7-(1-Aminomethyl-4-azatricyclo[5.2.2.0^{2,6}]undec-8-en-4-yl)-1-cyclopropyl-6,8-difluor-1,4-dihydro-4-oxo-3- chinolincarbonsäure,
Schmelzpunkt: 238-240°C (unter Zersetzung).

### Beispiel 30

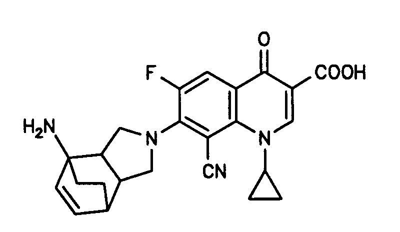

Entsprechend Beispiel 1 setzt man 8-Cyano-1-cyclopropyl-7-chlor-6-fluor-1,4-dihydro-4-oxo-3-chinolincarbonsäure ein und erhitzt während 8 Stunden auf 80°C. Nach Einengen und Aufarbeitung mit Ethanol erhält man in 51 %-iger Ausbeute 7-(1-Amino-4-azatricyclo[5.2.2.0^{2,6}]undec-8-en-4-yl)-8-cyano-1- cyclopropyl-6-fluor-1,4-dihydro-4-oxo-3-chinolincarbonsäure,
Schmelzpunkt 180-182°C (unter Zersetzung).

### Beispiel 31

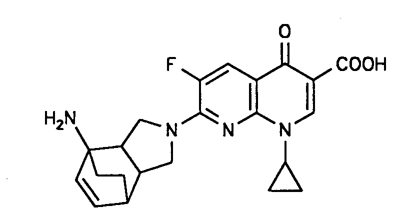

283 mg (1 mmol) 7-Chlor-1-cyclopropyl-6-fluor-1,4-dihydro-4-oxo-1,8-naphthyridin-3-carbonsäure werden in 6 ml Acetonitril bei 25°C mit 290 mg (≈ 1,5 mmol) 86 %-igem 4-Aza-tricyclo[5.2.2.0^{2,6}]undec-8-en-1-ylamin versetzt und 1 Stunde bei 25°C gerührt. Man saugt den Niederschlag ab, wäscht mit Ethanol, trocknet bei 60°C/0,1 mbar (Rohausbeute: 398 mg) und reinigt durch Chromatographie (Kieselgel, Dichlormethan/17 %-iges wäßriges Ammoniak/Methanol 30:8:1).
Ausbeute: 171 mg (42 % der Theorie) 7-(1-Amino-4-azatricyclo[5.2.2.0^{2,6}]undec-8-en-4-yl)-1-cyclopropyl-6-fluor- 1,4-dihydro-4-oxo-1,8-naphthyridin-3-carbonsäure,
Schmelzpunkt: 308-311°C (unter Zersetzung).

### Beispiel 32

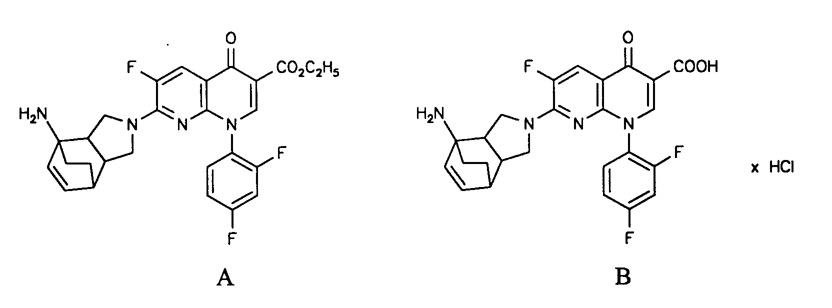

A. Analog Beispiel 31 wird mit 7-Chlor-6-fluor-1-(2,4-difluorphenyl)-1,4-dihydro-4-oxo-1,8-naphthyridin-3-carbonsäureethylester in 41 % Ausbeute zu 7-(1-Amino-4-azatricyclo[5.2.2.0^{2,6}]undec-8-en-4-yl)-6-fluor-1-(2,4-difluorphenyl)-1,4-dihydro-4-oxo-1,8-naphthyridin-3- carbonsäureethylester umgesetzt,
   Schmelzpunkt: 210-203°C (unter Zersetzung).
B. 170 mg (0,33 mmol) 7-(1-Amino-4-azatricyclo[5.2.2.0^{2,6}]undec-8-en-4-yl)-6-fluor-1-(2,4-difluorphenyl)-1,4-dihydro-4-oxo-1,8- naphthyridin-3-carbonsäureethylester werden in einer Mischung aus 2 ml Essigsäure und 1,5 ml halbkonzentrierter Salzsäure 2 Stunden unter Rückfluß erhitzt. Die Lösung wird eingeengt, mit wenig Wasser verrührt und der Niederschlag abgesaugt. Ausbeute: 150 mg (92 % der Theorie) 7-(1-Amino-4-azatricyclo[5.2.2.0^{2,6}]-undec-8-en-4-yl)-6-fluor-1-(2,4- difluorphenyl)-1,4-dihydro-4-oxo-1,8-naphthyridin-3-carbonsäure-Hydrochlorid,
   Schmelzpunkt: 270-272°C (unter Zersetzung).

### Beispiel 33

Unter entsprechenden Bedingungen wie in Beispiel 1 erhält man mit 6,7-Difluor-1-(2,4-difluorphenyl)-1,4-dihydro-5-methyl-4-oxo-3- chinolincarbonsäure ein Rohprodukt, das an Kieselgel chromatographisch gereinigt wird (Laufmittel: Dichlormethan/17 %-iges wäßriges Ammoniak/Methanol 30:8:1). Man isoliert in 91 %-iger Ausbeute 7-(1-Amino-4-azatricyclo[5.2.2.0^{2,6}]undec-8-en-4-yl)-6-fluor-1-(2,4-difluorphenyl)-1,4-dihydro-5-methyl-4-oxo-3- chinolincarbonsäure,
Schmelzpunkt: 281-283°C (unter Zersetzung).

### Beispiel 34

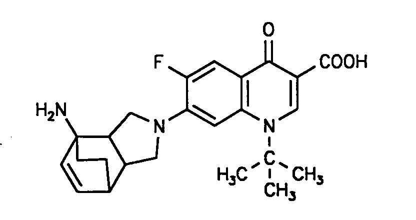

Unter entsprechenden Bedingungen wie in Beispiel 1 erhält man mit 1-tert.-Butyl-6,7-difluor-1,4-dihydro-4-oxo-3-chinolincarbonsäure in 61 %-iger Ausbeute 7-(1-Amino-4-aza-tricyclo[5.2.2.0^{2,6}]undec-8-en-4-yl)-1-tert.-butyl-6-fluor-1,4-dihydro-4-oxo-3-chinolincarbonsäure,
Schmelzpunkt: 263-265°C (unter Zersetzung).

## Patentansprüche

1. Verbindungen der Formel (I)
T-Q (I)
in welcher
Q einen Rest der Formeln 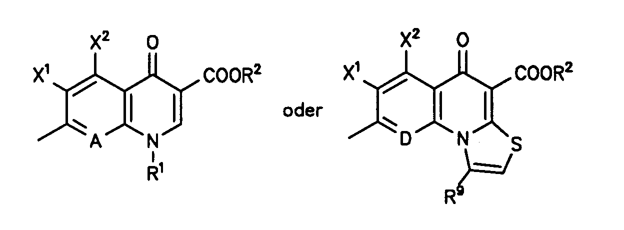 bedeutet, worin
R¹ für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
R² für Wasserstoff, gegebenenfalls durch Hydroxy, Methoxy, Amino, Dimethylamino substituiertes Alkyl mit 1 bis 3 Kohlenstoffatomen, Benzyl, (5-Methyl-2-oxo-1,3-dioxol-4-yl)-methyl, Acetoxymethyl, Pivaloyloxymethyl, 5-Indanyl, Phthalidinyl oder 3-Acetoxy-2-oxo-butyl,
R⁹ für Wasserstoff oder gegebenenfalls durch Methoxy, Hydroxy oder Halogen substituiertes Alkyl mit 1 bis 3 Kohlenstoffatomen,
X¹ für Halogen oder Nitro,
X² für Wasserstoff, Halogen, Amino, Hydroxy, Methoxy, Mercapto, Methyl, Halogenmethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-,-*S-CH₂-CH-CH₃,-*S-CH₂-CH-CH₂F, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff, Methyl oder Formyl bedeutet, bilden kann, und
D für N oder C-R¹⁰ steht, worin
R¹⁰ für Wasserstoff, Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(C₃H₅)-CH₂- oder -*S-CH₂-bilden kann, wobei das mit * markierte Atom mit dem Kohlenstoffatom von D verknüpft ist, und
T einen Rest der Formel 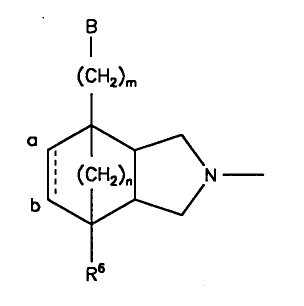 bedeutet, worin
B für NR³R⁴ oder OR⁵ steht, worin
R³ für Wasserstoff, Methyl oder Alkoxycarbonyl mit 1 bis 4 Kohlenstoffatomen im Alkylteil,
R⁴ für Wasserstoff oder Methyl und
R⁵ für Wasserstoff oder Methyl steht,
R⁶ für Wasserstoff oder Methyl,
m für 0 oder 1 und
n für 1 oder 2 steht,
wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali-, Silber- und Guanidiniumsalze der zugrundeliegenden Carbonsäuren.

2. Verbindungen der Formel (I) gemäß Anspruch 1,
in welcher
Q und T die oben angegebene Bedeutung haben und
R¹ für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
R² für Wasserstoff, Alkyl mit 1 bis 2 Kohlenstoffatomen, Benzyl oder (5-Methyl-2-oxo-1,3-dioxol-4-yl)-methyl,
R⁹ für Wasserstoff oder gegebenenfalls durch Fluor ein- bis dreifach substituiertes Alkyl mit 1 bis 2 Kohlenstoffatomen,
X¹ für Fluor oder Chlor,
X² für Wasserstoff, Halogen, Amino, Methyl, Trifluormethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet,
bilden kann, und
D für N oder C-R¹⁰ steht, worin
R¹⁰ für Wasserstoff, Fluor, Chlor, CF₃, OCH₃ oder CH₃ steht oder auch gemeinsam mit R⁹ eine Brücke der Struktur -O-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
B für NR³R⁴ oder OR⁵ steht, worin
R³ für Wasserstoff, Methyl oder Alkoxycarbonyl mit 1 bis 4 Kohlenstoffatomen im Alkylteil,
R⁴ für Wasserstoff oder Methyl und
R⁵ für Wasserstoff oder Methyl steht,
R⁶ für Wasserstoff oder Methyl,
m für 0 oder 1 und
n für 1 oder 2 steht,
wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali-, Silber- und Guanidiniumsalze der zugrundeliegenden Carbonsäuren.

3. Verbindungen der Formel (I) gemäß Anspruch 1,
in welcher
Q und T die oben angegebene Bedeutung haben und in welchen
R¹ für gegebenenfalls durch Fluor ein- oder zweifach substituiertes Alkyl mit 1 bis 4 Kohlenstoffatomen, Vinyl, gegebenenfalls durch 1 Fluor-atom substituiertes Cyclopropyl, gegebenenfalls durch Fluor ein-oder zweifach substituiertes Phenyl,
R² für Wasserstoff, Alkyl mit 1 bis 2 Kohlenstoffatomen oder (5-Methyl-2-oxo-1,3-dioxol-4-yl)methyl,
R⁹ für Wasserstoff oder gegebenenfalls durch Fluor ein- bis dreifach substituiertes Methyl,
X¹ für Fluor,
X² für Wasserstoff, Fluor, Amino, Methyl oder Vinyl,
A für N oder C-R⁷ steht, worin
R⁷ für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet,
bilden kann, und
D für N oder C-R¹⁰ steht, worin
R¹⁰ für Wasserstoff, Fluor, Chlor oder OCH₃ steht oder auch gemeinsam mit R⁹ eine Brücke der Struktur -*O-CH₂-, -*N(CH₃)-CH₂-, -*N(C₂H₅)-CH₂- oder -*S-CH₂- bilden kann, wobei das mit * markierte Atom mit dem Kohlenstoffatom von D verknüpft ist, und
B für NR³R⁴ oder OR⁵ steht, worin
R³ für Wasserstoff oder Methyl,
R⁴ für Wasserstoff oder Methyl und
R⁵ für Wasserstoff oder Methyl steht,
R⁶ für Wasserstoff,
m für 0 oder 1 und
n für 1 oder 2 steht,
wobei zwischen den Kohlenstoffatomen a und b eine Einfach- oder eine Doppelbindung stehen kann,
und deren pharmazeutisch verwendbaren Hydrate und Säureadditionssalze sowie die Alkali-, Erdalkali-, Silber- und Guanidiniumsalze der zugrundeliegenden Carbonsäuren.

4. Diastereomerenreine und enantiomerenreine Verbindungen nach den Ansprüchen 1 bis 3.

5. Verfahren zur Herstellung von Verbindungen der Formel (I) gemaß Ansprüchen 1 bis 4, dadurch gekennzeichnet, daß Verbindugnen der Formel (II)
Y-Q (II),
in welcher
Q die oben angegebene Bedeutung hat und
Y für Halogen, inbesondere für Fluor oder Chlor steht,
mit Verbindungen der Formel (III) 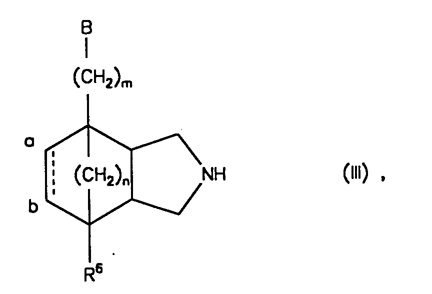 in welcher
B, R⁶, m, n, a und b die oben angegebenen Bedeutungen haben,
gegebenenfalls in Gegenwart von Säurefängern umgesetzt und gegebenenfalls vorhandene Schutzgruppen abgespalten werden.

6. Racemische, diastereomerenreine und enantiomerenreine Verbindungen aus der Gruppe bestehend aus
4-Azatricyclo[5.2.1.0^{2,6}]dec-8-en-1-ylamin,
4-Azatricyclo[5.2.2.0^{2,6}]undec-8-en-1-ylamin,
(4-Azatricyclo[5.2.2.0^{2,6}]undec-8-en-1-ylmethyl)amin,
(4-Azatricyclo[5.2.2.0^{2,6}]undec-8-en-1-yl)-dimethyl-amin,
4-Azatricyclo[5.2.2.0^{2,6}]undec-8-en-1-ol,
(4-Azatricyclo[5.2.2.0^{2,6}]undec-8-en-1-yl)-methanol,
4-Azatricyclo[5.2.1.0^{2,6}]dec-1-ylamin,
4-Azatricyclo[5.2.2.0^{2,6}]undec-1-ylamin.

7. Verbindungen gemäß Ansprüchen 1 bis 4 zur Bekämpfung von Krankheiten.

8. Verbindungen gemäß Ansprüchen 1 bis 4 zur Bekämpfung von bakteriellen Infektionen.

9. Verwendung von Verbindungen gemäß Ansprüchen 1 bis 4 bei der Herstellung von Arzneimitteln.

10. Arzneimittel enthaltend die Verbindungen gemäß Ansprüchen 1 bis 4.

11. Antibakterielle Mittel enthaltend Verbindungen gemäß Ansprüchen 1 bis 4.

## Claims

1. Compounds of the formula (I)
T-Q (I),
in which
Q denotes a radical of the formulae 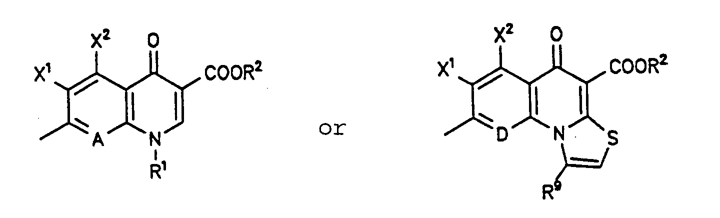 wherein
R¹ represents alkyl having 1 to 4 carbon atoms, which is optionally mono- or disubstituted by halogen or hydroxyl, alkenyl having 2 to 4 carbon atoms, cycloalkyl having 3 to 6 carbon atoms, which is optionally substituted by 1 or 2 fluorine atoms, bicyclo[1.1.1]pent-1-yl, 1,1-dimethylpropargyl, 3-oxetanyl, methoxy, amino, methylamino, dimethylamino, or phenyl which is optionally mono- or disubstituted by halogen, amino or hydroxyl,
R² represents hydrogen, alkyl having 1 to 3 carbon atoms, which is optionally substituted by hydroxyl, methoxy, amino or dimethylamino, benzyl, (5-methyl-2-oxo-1,3-dioxol-4-yl)-methyl, acetoxymethyl, pivaloyloxymethyl, 5-indanyl, phthalidinyl, 3-acetoxy-2-oxo-butyl,
R⁹ represents hydrogen or alkyl having 1 to 3 carbon atoms, which is optionally substituted by methoxy, hydroxy or halogen,
X¹ represents halogen or nitro,
X² represents hydrogen, halogen, amino, hydroxyl, methoxy, mercapto, methyl, halogenomethyl or vinyl,
A represents N or C-R⁷, wherein
R⁷ represents hydrogen, halogen, CF₃, OCH₃, OCHF₂, CH₃, CN, CH=CH₂ or C/CH or alternatively, together with R¹, can form a bridge of the structure -*O-CH₂-CH-CH₃, -*S-CH₂-CH₂-, -*S-CH₂-CH-CH₃, -*S-CH₂-CH-CH₂F, -*CH₂-CH₂-CH-CH₃ or -*O-CH₂-N-R⁸, the atom marked by * being linked to the carbon atom of A and wherein
R⁸ denotes hydrogen, methyl or formyl, and
D represents N or C-R¹⁰, wherein
R¹⁰ represents hydrogen, halogen, CF₃, OCH₃, OCHF₂ or CH₃ or alternatively, together with R⁹, can form a bridge of the structure -*O-CH₂- -*NH-CH₂- -*N(CH₃)-CH₂-, -*N(C₂H₅)-CH₂-, -*N(C₃H₅)-CH₂- or -*S-CH₂-, the atom marked by * being linked to the carbon atom of D, and
T denotes a radical of the formula 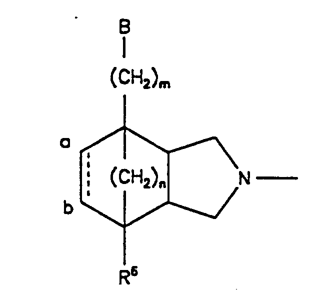 wherein
B represents NR³R⁴ or OR⁵, wherein
R³ represents hydrogen, methyl or alkoxycarbonyl having 1 to 4 carbon atoms in the alkyl moiety,
R⁴ represents hydrogen or methyl and
R⁵ represents hydrogen or methyl and
R⁶ represents hydrogen or methyl,
m represents 0 or 1 and
n represents 1 or 2,
it being possible for there to be a single or a double bond between the carbon atoms a and b,
and their pharmaceutically utilizable hydrates and acid addition salts and also the alkali metal, alkaline earth metal, silver and guanidinium salts of the underlying carboxylic acids.

2. Compounds of the formula (I) according to Claim 1,
in which
Q and T have the meaning indicated above and
R¹ represents alkyl having 1 to 4 carbon atoms, which is optionally mono- or disubstituted by halogen, alkenyl having 2 to 3 carbon atoms, cycloalkyl having 3 or 4 carbon atoms, which is optionally substituted by 1 fluorine atom, bicyclo[1.1.1]pent-1-yl, 1,1-dimethylpropargyl, 3-oxetanyl, methylamino, or phenyl which is optionally mono- or disubstituted by fluorine, amino or hydroxyl,
R² represents hydrogen, alkyl having 1 to 2 carbon atoms, benzyl or (5-methyl-2-oxo-1,3-dioxol-4-yl)-methyl,
R⁹ represents hydrogen or alkyl having 1 to 2 carbon atoms, which is optionally mono-to trisubstituted by fluorine,
X¹ represents fluorine or chlorine,
X² represents hydrogen, halogen, amino, methyl, trifluoromethyl or vinyl,
A represents N or C-R⁷, wherein
R⁷ represents hydrogen, halogen, CF₃, OCH₃, OCHF₂, CH₃, CN, CH=CH₂ or C/CH or alternatively, together with R¹, can form a bridge of the structure -*O-CH₂-CH-CH₃, -*S-CH₂-CH₂-, -^{*}CH₂-CH₂-CH-CH₃ or -*O-CH₂-N-R⁸, the atom marked by * being linked to the carbon atom of A and wherein
R⁸ denotes hydrogen or methyl, and
D represents N or C-R¹⁰, wherein
R¹⁰ represents hydrogen, fluorine, chlorine, CF₃, OCH₃ or CH₃ or alternatively, together with R⁹, can form a bridge of the structure O-CH₂-, -*N(CH₃)-CH₂- -*N(C₂H₅)-CH₂-, -*N(C₃H₅)-CH₂- or -*S-CH₂-, the atom marked by * being linked to the carbon atom of D, and
B represents NR³R⁴ or OR⁵, wherein
R³ represents hydrogen, methyl or alkoxycarbonyl having 1 to 4 carbon atoms in the alkyl moiety,
R⁴ represents hydrogen or methyl and
R⁵ represents hydrogen or methyl,
R⁶ represents hydrogen or methyl,
m represents 0 or 1 and
n represents 1 or 2,
it being possible for there to be a single or a double bond between the carbon atoms a and b,
and their pharmaceutically utilizable hydrates and acid addition salts and also the alkali metal, alkaline earth metal, silver and guanidinium salts of the underlying carboxylic acids.

3. Compounds of the formula (I) according to Claim 1,
in which
Q and T have the meaning indicated above, and in which
R¹ represents alkyl having 1 to 4 carbon atoms, which is optionally mono- or disubstituted by fluorine, vinyl, cyclopropyl which is optionally substituted by 1 fluorine atom, or phenyl which is optionally mono- or disubstituted by fluorine,
R² represents hydrogen, alkyl having 1 to 2 carbon atoms or (5-methyl-2-oxo-1,3-dioxol-4-yl)-methyl,
R⁹ represents hydrogen or methyl which is optionally mono- to trisubstituted by fluorine,
X¹ represents fluorine,
X² represents hydrogen, fluorine, amino, methyl or vinyl,
A represents N or C-R⁷, wherein
R⁷ represents hydrogen, fluorine, chlorine, bromine, CF₃, OCH₃, OCHF₂, CH₃, CN, CH=CH₂ or C/CH or alternatively, together with R¹, can form a bridge of the structure -*O-CH₂-CH-CH₃ or -*O-CH₂-N-R⁸, the atom marked by * being linked to the carbon atom of A and wherein
R⁸ denotes hydrogen or methyl,
and
D represents N or C-R¹⁰, wherein
R¹⁰ represents hydrogen, fluorine, chlorine or OCH₃ or alternatively, together with R⁹, can form a bridge of the structure -*O-CH₂-, -*N(CH₃)-CH₂-, -*N(C₂H₅)-CH₂- or -*S-CH₂-, the atom marked by * being linked to the carbon atom of D, and
B represents NR³R⁴ or OR⁵, in which
R³ represents hydrogen or methyl,
R⁴ represents hydrogen or methyl and
R⁵ represents hydrogen or methyl,
R⁶ represents hydrogen,
m represents 0 or 1 and
n represents 1 or 2,
it being possible for there to be a single or a double bond between the carbon atoms a and b,
and their pharmaceutically utilizable hydrates and acid addition salts and also the alkali metal, alkaline earth metal, silver and guanidinium salts of the underlying carboxylic acids.

4. Diasteromerically pure and enantiomerically pure compounds according to Claims 1 to 3.

5. Process for the preparation of compounds of the formula (I) according to Claims 1 to 4, characterized in that compounds of the formula (II)
Y-Q (II),
in which
Q has the meaning indicated above and
Y represents halogen, in particular fluorine or chlorine,
are reacted with compounds of the formula (III) 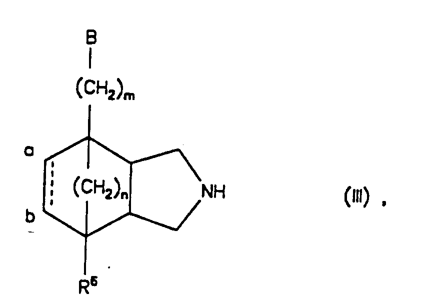 in which
B, R⁶, m, n, a and b have the meanings indicated above,
if appropriate in the presence of acid scavengers and, if appropriate, protective groups present are removed.

6. Racemic, diastereomericaily pure and enantiomerically pure compounds from the group consisting of
4-azatricyclo[5.2.1.0^{2,6}]dec-8-en-1-ylamine,
4-azatricyclo[5.2.2.0^{2,6}]undec-8-en-1-ylamine,
(4-azatricyclo[5.2.2.0^{2,6}]undec-8-en-1-ylmethyl)amine,
(4-azatricyclo[5.2.2.0^{2,6}]undec-8-en-1)-dimethylamine,
4-azatricyclo[5.2.2.0^{2,6}]undec-8-en-1-ol,
(4-azatricyclo[5.2.2.0^{2,6}]undec-8-en-1-yl)-methanol,
4-azatricyclo[5.2.2.0^{2,6}]dec-1-ylamine,
4-azatricyclo[5.2.2.0^{2,6}]undec-1-ylamine,

7. Compounds according to Claims 1 to 4 for the control of diseases.

8. Compounds according to Claims 1 to 4 for the control of bacterial infections.

9. Use of compounds according to Claims 1 to 4 in the production of medicaments.

10. Medicaments containing the compounds according to Claims 1 to 4.

11. Antibacterial compositions containing compounds according to Claims 1 to 4.

## Revendications

1. Composés de formule (I)
T-Q (I)
dans laquelle
Q est un reste de formules 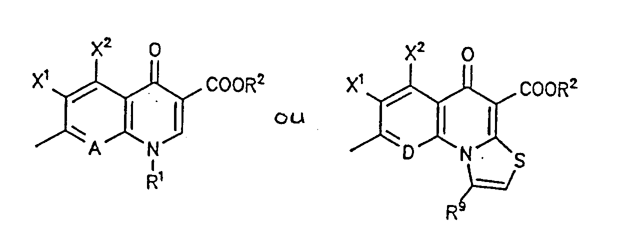 dans lesquelles .
R¹ est un groupe alkyle ayant 1 à 4 atomes de carbone éventuellement substitué une ou deux fois par un halogène ou un radical hydroxy, un groupe alcényle ayant 2 à 4 atomes de carbone, un groupe cycloalkyle ayant 3 à 6 atomes de carbone éventuellement substitué par un ou deux atomes de fluor, un groupe bicyclo[1.1.1]pent-1-yle, 1,1-diméthylpropargyle, 3-oxétannyle, méthoxy, amino, méthylamino, diméthylamino, un groupe phényle éventuellement substitué une ou deux fois par un halogène, un radical amino ou hydroxy,
R² représente l'hydrogène, un groupe alkyle ayant 1 à 3 atomes de carbone éventuellement substitué par un radical hydroxy, méthoxy, amino, diméthylamino, un groupe benzyle, un groupe (5-méthyl-2-oxo-1,3-dioxole-4-yl)-méthyle, acétoxyméthyle, pivaloyloxyméthyle, 5-indanyle, phtalidinyle ou 3-acétoxy-2-oxobutyle,
R⁹ représente l'hydrogène ou un groupe alkyle ayant 1 à 3 atomes de carbone éventuellement substitué par un radical méthoxy, hydroxy ou un halogène,
X¹ est un halogène ou un groupe nitro,
X² représente l'hydrogène, un halogène, un groupe amino, hydroxy, méthoxy, mercapto, méthyle, halogénométhyle ou vinyle,
A représente N ou un groupe C-R⁷ dans lequel
R⁷ est l'hydrogène, un halogène, un groupe CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂CH₂-, -*S-CH₂-CH-CH₃, -*S-CH₂-CH-CH₂F, -*CH₂-CH₂-CH-CH₃ ou -*O-CH₂-N-R⁸, où l'atome marqué d'un * est lié à l'atome de carbone de A et où
R⁸ représente l'hydrogène, un groupe méthyle ou formyle,
et
D représente N ou un groupe C-R¹⁰, dans lequel
R¹⁰ est l'hydrogène, un halogène, un groupe CF₃, OCH₃, OCHF₂ ou CH₃ ou peut aussi former conjointement avec R⁹ un pont de structure -*O-CH₂-, -*NH-CH₂-, -*N(CH₃)-CH₂-, -*N(C₂H₅)-CH₂-, -*N(C₃H₅)-CH₂- ou -*S-CH₂-, où l'atome marqué d'un * est lié à l'atome de carbone de D, et
T représente un reste de formule 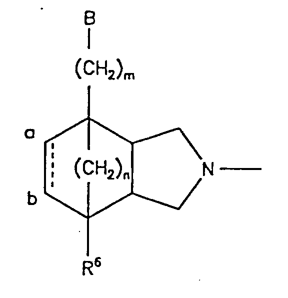 dans laquelle
B est un groupe NR³R⁴ ou OR⁵, où
R³ représente l'hydrogène, un groupe méthyle ou un groupe alkoxycarbonyle ayant là 4 atomes de carbone dans la partie alkyle,
R⁴ représente l'hydrogène ou un groupe méthyle, et
R⁵ représente l'hydrogène ou un groupe méthyle,
R⁶ est l'hydrogène ou un groupe méthyle,
m a la valeur 0 ou 1 et
n a la valeur 1 ou 2,
une liaison simple ou une liaison double pouvant exister entre les atomes de carbone a et b,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

2. Composés de formule (I) suivant la revendication 1,
dans laquelle
Q et T ont la définition indiquée ci-dessus, et
R¹ représente un groupe alkyle ayant 1 à 4 atomes de carbone éventuellement substitué une ou deux fois par un halogène, un groupe alcényle ayant 2 ou 3 atomes de carbone, un groupe cycloalkyle de 3 ou 4 atomes de carbone éventuellement substitué par un atome de fluor, un groupe bicyclo[1.1.1]pent-1-yle, 1,1-diméthylpropargyle, 3-oxétannyle, méthylamino, un groupe phényle éventuellement substitué une ou deux fois par du fluor, un radical amino ou hydroxy,
R² représente l'hydrogène, un groupe alkyle ayant 1 ou 2 atomes de carbone, benzyle ou (5-méthyl-2-oxo-1,3-dioxole-4-yl)-méthyle,
R⁹ représente l'hydrogène ou un groupe alkyle ayant 1 ou 2 atomes de carbone éventuellement substitué une à trois fois par du fluor,
X¹ représente le fluor ou le chlore,
X² représente l'hydrogène, un halogène, un groupe amino, méthyle, trifluorométhyle ou vinyle,
A représente N ou un groupe C-R⁷ dans lequel
R⁷ est l'hydrogène, un halogène, un groupe CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂CH-, -*CH₂-CH₂-CH-CH₃ ou -*O-CH₂-N-R⁸, où l'atome marqué d'un * est lié à l'atome de carbone de A et où
R⁸ représente l'hydrogène ou le groupe méthyle, et
D représente N ou un groupe C-R¹⁰, dans lequel
R¹⁰ est l'hydrogène, le fluor, le chlore, un groupe CF₃, OCH₃ ou CH₃ ou peut aussi former conjointement avec R⁹ un pont de structure -O-CH₂-, -*N(CH₃)-CH₂-, -*N(C₂H₅)-CH₂, -*N(C₃H₅)-CH₂- ou -*S-CH₂-, où l'atome marqué d'un * est lié à l'atome de carbone de D, et
B est un groupe NR³R⁴ ou OR⁵, dans lequel
R³ représente l'hydrogène, un groupe méthyle ou un groupe alkoxycarbonyle ayant 1 à 4 atomes de carbone dans la partie alkyle,
R⁴ est l'hydrogène ou le groupe méthyle et
R⁵ est l'hydrogène ou le groupe méthyle,
R⁶ est l'hydrogène ou le groupe méthyle,
m a la valeur 0 ou 1 et
n a la valeur 1 ou 2,
une liaison simple ou une liaison double pouvant exister entre les atomes de carbone a et b,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

3. Composés de formule (I) suivant la revendication 1,
dans laquelle
Q et T ont la définition indiquée ci-dessus et dans lesquels
R¹ est un groupe alkyle ayant 1 à 4 atomes de carbone éventuellement substitué une ou deux fois par du fluor, un groupe vinyle, un groupe cyclopropyle éventuellement substitué par un atome de fluor, un groupe phényle éventuellement substitué une ou deux fois par du fluor,
R² représente l'hydrogène, un groupe alkyle ayant 1 ou 2 atomes de carbone ou le groupe (5-méthyl-2-oxo-1,3-dioxole-4-yl)-méthyle,
R⁹ est l'hydrogène ou un groupe méthyle éventuellement substitué une à trois fois par du fluor,
X¹ est le fluor,
X² est l'hydrogène, le fluor, un groupe amino, méthyle ou vinyle,
A représente N ou un groupe C-R⁷, dans lequel
R⁷ est l'hydrogène, le fluor, le chlore, le brome, un groupe CF₃, OCH₃, OCHF₂, CH₃, CN, CH=CH₂ ou C≡CH ou peut aussi former conjointement avec R¹ un pont de structure -*O-CH₂-CH-CH₃ ou -*O-CH₂-N-R⁸, où l'atome marqué d'un * est lié à l'atome de carbone de A et où
R⁸ représente l'hydrogène ou le groupe méthyle, et
D représente N ou un groupe C-R¹⁰, dans lequel
R¹⁰ est l'hydrogène, le fluor, le chlore ou un groupe OCH₃ ou peut aussi former conjointement avec R⁹ un pont de structure -*O-CH₂-, -*N(CH₃)-CH₂-, -*N-(C₂H₅)-CH₂- ou -*S-CH₂-, où l'atome marqué d'un * est lié à l'atome de carbone de D, et
B représente un groupe NR³R⁴ ou OR⁵, dans lequel R³ est l'hydrogène ou le groupe méthyle, R⁴ est l'hydrogène ou le groupe méthyle, et R⁵ est l'hydrogène ou le groupe méthyle,
R⁶ est l'hydrogène,
m a la valeur 0 ou 1 et
n a la valeur 1 ou 2,
une liaison simple ou une liaison double pouvant exister entre les atomes de carbone a et b,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

4. Composés diastéréoisomères purs et énantiomères purs selon les revendication 1 à 3.

5. Procédé de production de composés de formule (I) suivant les revendications 1 à 4, caractérisé en ce qu'on fait réagir des composés de formule (II)
Y-Q (II)
dans laquelle
Q a la définition indiquée ci-dessus et
Y représente un halogène, en particulier le fluor ou le chlore,
avec des composés de formule (III) 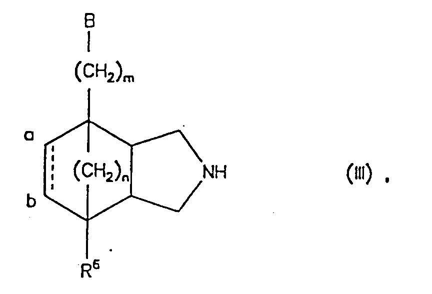 dans laquelle
B, R⁶, m, n, a et b ont les définitions indiquées ci-dessus,
le cas échéant en présence d'accepteurs d'acides et on élimine les groupes protecteurs éventuellement présents.

6. Composés racémiques, diastéréoisomères purs et énantiomères purs du groupe constitué de :
la 4-azatricyclo[5.2.1.0^{2,6}]déc-8-ène-1-ylamine
la 4-azatricyclo[5.2.2.0^{2,6}]undéc-8-ène-1-ylamine,
la (4-azatricyclo[5.2.2.0^{2,6}]undéc-8-ène-1-ylméthyl)amine,
la (4-azatricyclo[5.2.2.0^{2,6}]undéc-8-ène-1-yl)diméthylamine,
le 4-azatricyclo[5.2.2.0^{2,6}]undéc-8-ène-1-ol,
le (4-azatricyclo[5.2.2.0^{2,6}]undéc-8-ène-1-yl)-méthanol,
la 4-azatricyclo[5.2.1.0^{2,6}]déc-1-ylamine,
la 4-azatricyclo[5.2.2.0^{2,6}]undéc-1-ylamine.

7. Composés suivant les revendications 1 à 4, destinés à combattre des maladies.

8. Composés suivant les revendications 1 à 4, destinés à combattre des infections bactériennes.

9. Utilisation de composés suivant les revendications 1 à 4 dans la préparation de médicaments.

10. Médicaments contenant les composés suivant les revendications 1 à 4.

11. Compositions antibactériennes contenant des composés suivant les revendications 1 à 4.
